# EUROPEAN PATENT APPLICATION

(11) **EP 4 205 764 A1**
(43) Date of publication of application: **05.07.2023**
(21) Application number: 21861669.6
(22) Date of filing: 26.08.2021
(51) Int. Cl.: A61K 39/395, A61P 35/00, A61P 35/02, C07K 16/18, C07K 16/28, C07K 16/46, C12N 15/13, A61K 51/10, G01N 33/53, G01N 33/531

(54) **ANTI-TRUNCATED MUTANT CALR-CD3 BISPECIFIC ANTIBODY AND PHARMACEUTICAL COMPOSITION**

(30) Priority: 27.08.2020 JP 2020143551
(71) Applicant: Juntendo Educational Foundation, Tokyo 113-8421 (JP); Meiji Seika Pharma Co., Ltd., Tokyo 104-8002 (JP)
(72) Inventor: KOMATSU, Norio, Tokyo 113-8421 (JP); ARAKI, Marito, Tokyo 113-8421 (JP); KIHARA, Yoshihiko, Tokyo 113-8421 (JP); ISHIDA, Yoji, Tokyo 104-8002 (JP); KITAMURA, Koichi, Tokyo 104-8002 (JP); FUKUSHIMA, Takayoshi, Odawara-shi, Kanagawa 250-0852 (JP); YASUI, Kaori, Tokyo 104-8002 (JP)
(74) Representative: Plougmann Vingtoft a/s
(86) International application number: PCT/JP2021/031355
(87) International publication number: WO 2022/045247

(57) **Abstract**

Provided is a higher performance bispecificity antibody which specifically binds to a mutant calreticulin protein and a pharmaceutical composition comprising the antibody. Provided is the bispecific antibody having a first domain which specifically binds to the mutant calreticulin protein and a second domain which specifically binds to a CD3 antigen.

## Description

### Technical Field

The present invention relates to the field of a bispecific antibody having specificity for a mutant CALR protein and its utilization in therapeutic and diagnostic applications.

### Background Art

In a part of patients with Philadelphia-negative myeloproliferative neoplasms (MPNs), nucleotide deletion or insertion is found in exon 9 of the calreticulin (CALR) gene (Non Patent Literatures 1 and 2). It has been already revealed that the mutant CALR protein produced by a mutant CALR gene has oncogenicity independently causing a myeloproliferative neoplasm (MPN) by constitutively activating a thrombopoietin (TPO) receptor (Non Patent Literatures 3 to 6).

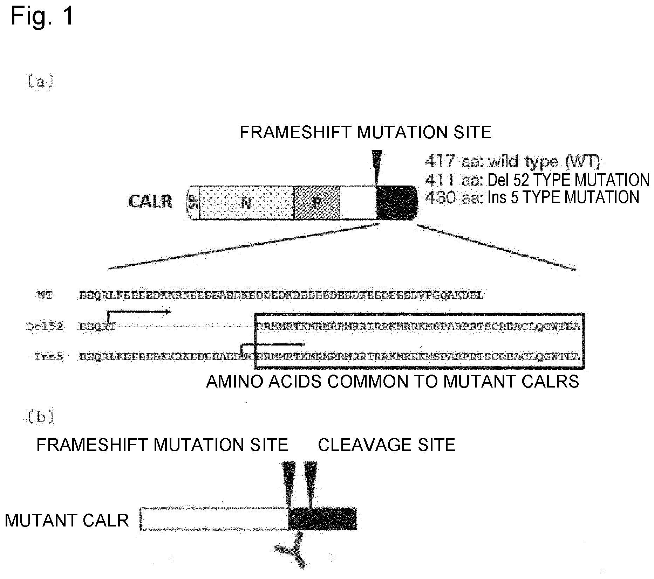

The CALR gene mutation found in MPN patients is a frameshift mutation which is always localized in the last exon, and a shift in the amino acid reading frame due to the frameshift mutation is always +1. From these findings, a sequence not present in the wild type is present at the C-terminus of the mutant CALR protein, and particularly, 44 amino acids at the C-terminus are common to almost all mutant CALR proteins. As an example thereof, Figure 1 [a] shows a comparison of the C-terminal sequences of 52-nucleotide deletion type (Del 52), which is the most frequent mutation among CALR gene mutations found in MPN patients, and 5-nucleotide insertion type (Ins 5), which is the next most frequent CALR mutation, with the corresponding region of the wild-type CALR protein.

Since the mutant CALR protein causing an MPN is expressed in tumor cells, it has been suggested that a sequence specific to the mutant CALR protein caused by the frameshift mutation may become a diagnostic marker or a therapeutic target as a neoantigen (Patent Literatures 1 and 2).

However, it turned out that an antibody that simply recognizes a sequence specific to the mutant CALR protein (neoantigen) generated by the frameshift mutation cannot accurately detect the mutant CALR protein in a cell extract or on a cell surface. As a result of functional analysis of the mutant CALR proteins, many of the mutant CALR proteins expressed in tumor cells are cleaved in sequences specific to the mutant CALR proteins and that cleaved mutant CALR proteins, in which most of the sequences inferred as neoantigens have been lost, are largely expressed. Accordingly, when an antibody which specifically recognizes a very short amino acid sequence located at the N-terminal side of the cleavage site in the neoantigen was made (Figure 1 [b]), it was revealed that an excellent MPN therapeutic effect is obtained. From these findings, it was suggested that, in diagnosis or treatment of MPN patients having a CALR gene mutation, pharmaceutical products that achieve higher-performance can be developed by detecting or targeting mutant CALR proteins including not only the full-length (uncleaved) but also cleaved proteins.

Furthermore, in recent years, an antibody capable of specifically binding to two antigens for the purpose of enhancing anticancer activity has been actively studied as a more effective therapeutic agent with specific antitumor effects by taking advantage of the properties of the antibody. Much attention has been paid to a bispecific antibody in which one site can bind to a surface antigen on a target cell and the other site can bind to a surface antigen on an effector cell. As the effector cell, an immune effector cell such as a T cell is often used, and the bispecific antibody aims to bring about a transient interaction between the target cell and the immune effector cell. Among T cell surface antigens, for example, an antibody having a binding site for a CD3 epsilon subunit derived from a T-cell receptor (TCR) protein complex is capable of causing lysis of the target cell primarily by the T cell, that is, T cell-redirected killing. Thus, since the bispecific antibody specifically binds to a target cell and a T cell and causes the T cell to directly act on the target cell, studies are in progress to target substances abnormally expressed in cancer cells or substances altered during oncogenic transformation.

For example, Patent Literature 3 describes a bispecific antibody with the first specificity for human CD19 and the second specificity for a human CD3 antigen, and Patent Literature 4 describes a bispecific antibody related to CALR and immune cells.

### Citation List

### Patent Literature

Patent Literature 1: WO 2015/036599
Patent Literature 2: WO 2016/087514
Patent Literature 3: WO 1999/054440
Patent Literature 4: WO 2019/178362

### Non Patent Literature

Non Patent Literature 1: Klampfl T, Gisslinger H, Harutyunyan AS, et al. Somatic mutations of calreticulin in myeloproliferative neoplasms. The New England journal of medicine. 2013; 369: 2379-90.
Non Patent Literature 2: Nangalia J, Massie CE, Baxter EJ, et al. Somatic CALR mutations in myeloproliferative neoplasms with nonmutated JAK2. The New England journal of medicine. 2013; 369: 2391-405.
Non Patent Literature 3: Araki M, Yang Y, Masubuchi N, et al. Activation of the thrombopoietin receptor by mutant calreticulin in CALR-mutant myeloproliferative neoplasms. Blood. 2016; 127: 1307-16.
Non Patent Literature 4: Elf S, Abdelfattah NS, Chen E, et al. Mutant Calreticulin Requires Both Its Mutant C-terminus and the Thrombopoietin Receptor for Oncogenic Transformation. Cancer Discov. 2016; 6: 368-81.
Non Patent Literature 5: Marty C, Pecquet C, Nivarthi H, et al. Calreticulin mutants in mice induce an MPL-dependent thrombocytosis with frequent progression to myelofibrosis. Blood. 2016; 127: 1317-24.
Non Patent Literature 6: Vainchenker W, Kralovics R. Genetic basis and molecular pathophysiology of classical myeloproliferative neoplasms. Blood. 2017; 129: 667-79.

### Summary of Invention

### Technical Problem

Although mutant CALR proteins are specifically expressed in MPN patient cells, many of the mutant CALR proteins were cleaved mutant CALR proteins. Therefore, the problem of the present invention is to provide a pharmaceutical composition containing an antibody or functional fragment thereof which is capable of accurately detecting the full-length and cleaved mutant CALR proteins and can be therapeutically applied.

### Solution to Problem

By using an antibody which specifically recognizes a very short amino acid sequence located at the N-terminal side of the cleavage site, it was verified that the cleaved mutant CALR protein was expressed not only in cultured cells but also in, for example, patient peripheral blood cells, patient platelets, and patient bone marrow cell. When using this anti-cleaved mutant CALR antibody, the detection sensitivity for the mutant CALR protein on a cell surface was dramatically improved. Furthermore, it was revealed that when a bispecific antibody (anti-cleaved mutant CALR-CD3 bispecific antibody) containing a protein which binds to a cleaved mutant CALR protein and CD3 antigen was made, excellent therapeutic effects are obtained against MPNs. It was found that a bispecific antibody based on an antibody to sequences conventionally considered as neoantigens did not provide sufficient therapeutic effects, strongly suggesting that it is important to provide a higher-performance bispecific antibody which specifically binds to the full-length and cleaved mutant CALR protein and a pharmaceutical composition containing the antibody, thus completing the present invention.

Accordingly, the present invention provides the following (1) to (22).
(1) A bispecific antibody comprising a first domain which specifically binds to a mutant calreticulin protein and a second domain which specifically binds to a CD3 antigen.
(2) The bispecific antibody according to (1), wherein the first domain competes with an antibody which binds to an epitope of a mutant calreticulin protein of SEQ ID NO: 1.
(3) The bispecific antibody according to (1) or (2), wherein the mutant calreticulin protein comprises an antigen-recognition site in a polypeptide chain consisting of the amino acid sequence of SEQ ID NO: 1 or a polypeptide chain consisting of the amino acid sequence of SEQ ID NO: 1 but having deletion, substitution, or addition of one or several amino acids.
(4) The bispecific antibody according to any one of (1) to (3), wherein the first domain does not bind to a wild-type calreticulin protein but has high affinity to the mutant calreticulin protein.
(5) The bispecific antibody according to any one of (1) to (4), wherein the first domain comprises a high affinity to the mutant calreticulin protein after cleavage.
(6) The bispecific antibody according to any one of (1) to (5), wherein a binding of the first domain with the mutant calreticulin protein has a K_{D} of 10⁻⁷ M or less.
(7) The bispecific antibody according to any one of (1) to (6), wherein a CDR of the first domain comprises (a) an amino acid sequence having 80% or more homology to the amino acid sequences of SEQ ID NO: 10, SEQ ID NO: 11 or 126, and SEQ ID NO: 12 (VHCDR), and an amino acid sequence having 80% or more homology to the amino acid sequences of SEQ ID NO: 13, SEQ ID NO: 14 or 127, and SEQ ID NO: 15 (VLCDR); (b) an amino acid sequence having 80% or more homology to the amino acid sequences of SEQ ID NO: 16 to 18 (VHCDR), and an amino acid sequence having 80% or more homology to the amino acid sequences of SEQ ID NO: 19 to 21 (VLCDR); or (c) an amino acid sequence having 80% or more homology to the amino acid sequences of SEQ ID NO: 22 to 24 (VHCDR), and an amino acid sequence having 80% or more homology to the amino acid sequences of SEQ ID NO: 25 to 27 (VLCDR).
(8) The bispecific antibody according to any one of (1) to (7), wherein the first domain comprises (d) a VH region having an amino acid sequence having 80% or more homology to the amino acid sequences of SEQ ID NO: 2, 90, 91, 92, 93, 94, 133, 134, 135, and 136, and a VL region having an amino acid sequence having 80% or more homology to the amino acid sequences of SEQ ID NO: 3, 95, 96, 97, 98, 99, 137, 138, 139, 140, and 141; (e) a VH region having an amino acid sequence having 80% or more homology to the amino acid sequence of SEQ ID NO: 4, and a VL region having an amino acid sequence having 80% or more homology to the amino acid sequence of SEQ ID NO: 5; or (f) a VH region having an amino acid sequence having 80% or more homology to the amino acid sequence of SEQ ID NO: 6, and a VL region having an amino acid sequence having 80% or more homology to the amino acid sequence of SEQ ID NO: 7.
(9) The bispecific antibody according to any one of (1) to (8), wherein a CDR of the second domain comprises an amino acid sequence having 80% or more homology to the amino acid sequences of SEQ ID NO: 28, SEQ ID NO: 29 or 128, and SEQ ID NO: 30, 129 or 130 (VHCDR), and an amino acid sequence having 80% or more homology to the amino acid sequences of SEQ ID NO: 31 or 131, SEQ ID NO: 32 or 132, and SEQ ID NO: 33 (VLCDR).
(10) The bispecific antibody according to any one of (1) to (9), wherein the second domain comprises a VH region having an amino acid sequence having 80% or more homology to the amino acid sequences of SEQ ID NO: 8, 204, 205, and 206, and a VL region having an amino acid sequence having 80% or more homology to the amino acid sequences of SEQ ID NO: 9, 207, and 208.
(11) The bispecific antibody according to any one of (1) to (10), wherein a dimerization module is a hetero-H chain having one or more knob-into-hole structures, wherein an amino acid(s) at one or more positions selected from the group consisting of 349, 354, 366, 368, and 407 in a constant region of the H chain are replaced by Y349C, T366S, L368A, Y407V (hole or cavity)S354C, or T366W (knob or protrusion), the bispecific antibody comprising an Fc region of SEQ ID NO:209 or 210.
(12) The bispecific antibody according to any one of (1) to (11), comprising a mutated Fc region of SEQ ID NO: 211 with substitution of an amino acid(s) at one or more positions selected from the group consisting of 234 and 235 in a H-chain Fc region to each introduce L234A or L235A mutation.
(13) The bispecific antibody according to any one of (1) to (12), which is a human antibody, a humanized antibody, a chimeric antibody of an animal-derived antibody and a human antibody, or a synthetic antibody.
(14) A pharmaceutical composition comprising the bispecific antibody according to any one of (1) to (13) or a functional fragment thereof.
(15) The pharmaceutical composition according to (14), for treatment or diagnosis of a myeloproliferative neoplasm.
(16) A method for detecting a mutant calreticulin protein, comprising detecting the following polypeptide (A) or (B) in a biological sample using the bispecific antibody according to any one of (1) to (13) or a functional fragment thereof:
   (A) a polypeptide consisting of the amino acid sequence of SEQ ID NO: 1; or
   (B) a polypeptide consisting of an amino acid sequence of SEQ ID NO: 1 but having deletion, substitution, or addition of one or several amino acids.
(17) The bispecific antibody according to any one of (1) to (13) or a functional fragment thereof, for use in detection of a mutant calreticulin protein.
(18) A diagnostic method for a myeloproliferative neoplasm, comprising detecting the following polypeptide (A) or (B) in a biological sample using the bispecific antibody according to any one of (1) to (13) or a functional fragment thereof:
   (A) a polypeptide consisting of the amino acid sequence of SEQ ID NO: 1; or
   (B) a polypeptide consisting of an amino acid sequence of SEQ ID NO: 1 but having deletion, substitution, or addition of one or several amino acids.
(19) The bispecific antibody according to any one of (1) to (13) or a functional fragment thereof, for use in treatment or diagnosis of a myeloproliferative neoplasm.
(20) Use of the bispecific antibody according to any one of (1) to (13) or a functional fragment thereof, for producing a medicament for treatment or diagnosis of a myeloproliferative neoplasm.
(21) A method for prevention or treatment of a tumor, comprising administering the bispecific antibody according to any one of (1) to (13) or a functional fragment thereof.
(22) The method according to (21), wherein the tumor is a hematologic tumor, and preferably a myeloproliferative neoplasm, myeloid leukemia, thrombocytosis, polycythemia, primary myelofibrosis, or secondary myelofibrosis.

### Advantageous Effects of Invention

The bispecific antibody of the present invention has an antigen-recognition site (epitope) in the polypeptide chain (A) or (B), and by using this antibody, it is possible to detect a cleaved mutant CALR protein caused by cleavage of a mutant CALR protein and to dramatically improve the detection sensitivity for the mutant CALR protein on a cell surface. The development of anti-cleaved mutant CALR-CD3 bispecific antibodies having the first specificity for binding to the epitope of the cleaved mutant CALR protein and the second specificity for binding to CD3 antigen will provide an antibody having strong cytotoxic activity specific to mutant CALR positive cells. Therefore, the bispecific antibody of the present invention can be used to specifically prevent or treat diseases with expression of a mutant CALR.

### Brief Description of Drawings

[Figure 1] Figure 1 is a diagram showing the characteristics of mutant CALR proteins. [a] The CALR gene mutation responsible for the development in MPN patients is +1 frameshift mutation, and an amino acid sequence common to mutant proteins is found in the C-terminus of the mutant CALR proteins produced as the results of the mutation. SP: signal sequence, N: N domain, and P: P domain. The arrow indicates where the amino acid sequences different from the wild-type (WT) sequence start. [b] An antibody which recognizes a mutant-specific sequence which is N-terminal to the cleavage site of the protein occurring within the mutant CALR protein-specific sequence is shown (black).

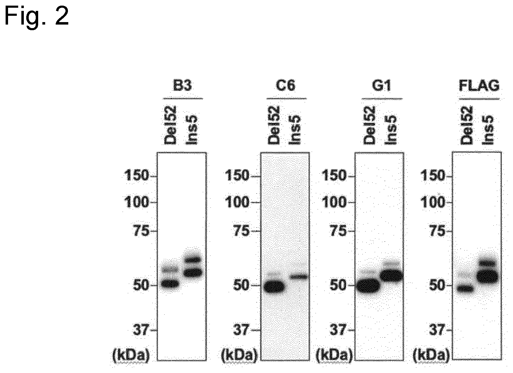

[Figure 2] Figure 2 shows that all clone B3, C6, and G1 antibodies recognize the full-length and cleaved mutant CALR proteins. In detail, the diagram shows the results of immunoblot analysis of proteins secreted from UT-7/TPO cells using the clone B3, C6, and G1 antibodies. Both the full-length Del 52 type and the Ins 5 type each with a FLAG-tag added to the downstream of the N-terminal signal sequence and the cleaved CALR proteins having lower molecular weights are also detected by any of the B3, C6, and G1 antibodies, as well as the anti-FLAG antibody.

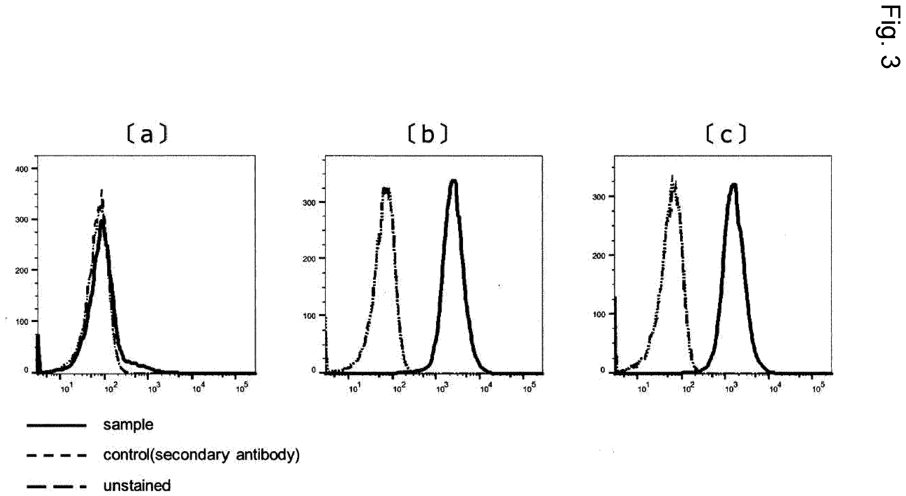

[Figure 3] Figure 3 shows the results of flow cytometry analysis of [a] UT-7/TPO vec cells expressing no mutant CALR, [b] cells expressing Ins 5 mutant CALR (UT-7/TPO CALR Ins 5 cells), or [c] cells expressing Del 52 mutant CALR (UT-7/TPO CALR Del 52 cells) using an anti-cleaved mutant CALR-CD3 bispecific antibody made using a VH chain of SEQ ID NO: 2, a VL chain of SEQ ID NO: 3, a VH chain of SEQ ID NO: 8, and a VL chain of SEQ ID NO: 9.

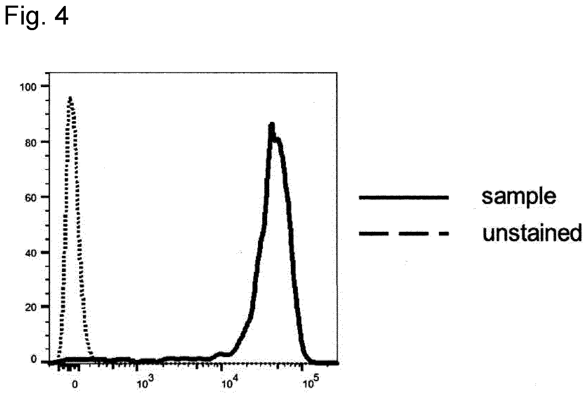

[Figure 4] Figure 4 shows the results of flow cytometry analysis of CD3 expressing cells (T-LAK cells) using the anti-cleaved mutant CALR-CD3 bispecific antibody described in Figure 3.

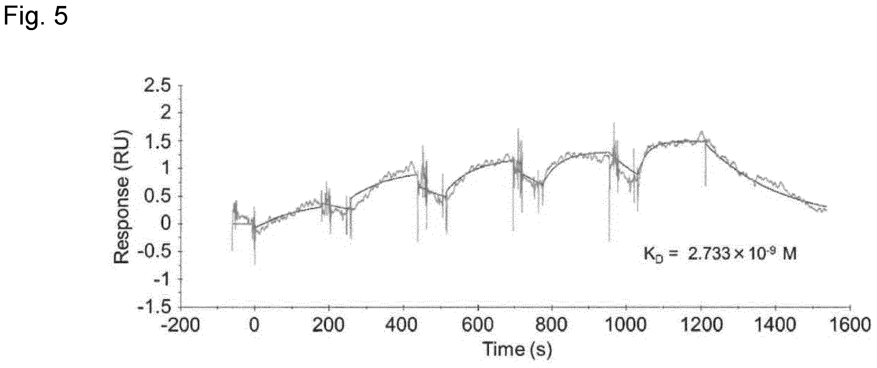

[Figure 5] Figure 5 shows the results of the evaluation, by the surface plasmon resonance analysis method, of binding strength to the anti-cleaved mutant CALR-CD3 bispecific antibody described in Figure 3 and antigen used to make the antibody.

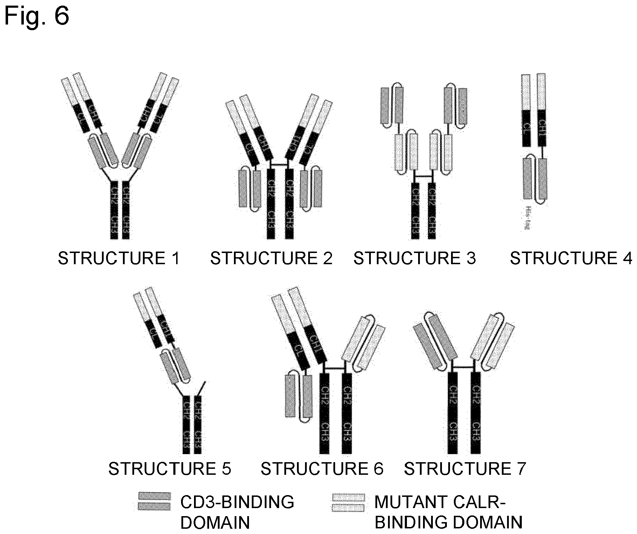

[Figure 6] Figure 6 is a schematic diagram showing an anti-cleaved CALR-CD3 bispecific antibody.

### Description of Embodiments

The present invention is directed to a bispecific antibody which recognizes only 13 amino acids (SEQ ID NO: 1: RRMMRTKMRMRRM) in the C-terminal polypeptide (Figure 1[a]) which remains after cleavage in a sequence specific to a mutant CALR protein, used in methods for detecting and diagnosing mutant CALR proteins related to an MPN and for prevention or treatment of an MPN, and the present inventors have found for the first time that the C-terminal side of the mutant CALR protein is cleaved, resulting in a cleaved mutant CALR protein, and that most of the common region of the mutant CALR protein is lost from the mutant CALR protein. Furthermore, the present inventors have found that a bispecific antibody with a first domain which binds to the cleaved mutant CALR protein and a second domain which binds to the CD3 antigen can be effectively used to treat, prevent, detect, and diagnose tumors.

In other words, the present invention provides a bispecific antibody which specifically recognizes the cleaved mutant CALR protein and the CD3 antigen, and a pharmaceutical composition containing the bispecific antibody, especially a pharmaceutical composition for treatment or prevention of an MPN.

As used herein, "bispecific antibody" refers to a monoclonal antibody having at least two antigen-binding sites capable of binding to different antigens. Specifically, the "bispecific antibody" means proteins having the ability to recognize two different antigens, which includes, for example, at least one first antigen-binding site (first domain) formed from the variable region of the heavy chain of the first antibody and the variable region of the light chain of the first antibody, and at least one second antigen-binding site (second domain) formed from the variable region of the heavy chain of the second antibody and the variable region of the light chain of the second antibody.

The form of the bispecific antibody is not particularly limited and may be any form known in the art, or may be any form as long as it retains specificity for the two antigens. The form of the bispecific antibody is broadly classified into IgG-like type and low molecular weight type. An IgG-like type is a form that retains the Fc region. Examples of the forms of IgG-like antibodies include, but are not limited to, CrossMab, DAF (two-in-one), DAF (four-in-one), DutaMab, DT-IgG, knobs-into-holes, knobs-into-holes common LC, SEEDbody, Triomab, κλ-body, DVD-Ig, IgG-scFv, and DuoBody. Since the IgG-like antibodies retain the Fc region, they are expected to, for example, exhibit effector functions such as ADCC and CDC, facilitate purification, improve stability, and extend the half-life in the blood. Meanwhile, the low molecular weight type usually has an Fv region composed of a heavy chain variable region and a light chain variable region as its basic component. Examples of the forms of the low molecular weight antibodies include, but are not limited to, Diabody (Db), BiTE, DART, TandAb, scDb, triple body, mini antibody, minibody, scFv, tandem scFv, and F(ab')2. The low molecular weight type is expected to have improved tissue permeability and high productivity due to its size. In addition, modified antibodies are also included, such as those in which amino acid sequence is deleted, substituted, or added while retaining the ability to bind to an antigen, those in which part or all of the sugar chain is deleted or added, those in which a linker or the like is added, those fused with other proteins, and antibody-drug conjugates (ADC) in which an antibody is bound to a low molecular weight pharmaceutical via a linker.

The bispecific antibody of the present invention has a first domain which specifically binds to a mutant calreticulin protein and a second domain which specifically binds to a CD3 antigen.

The first domain competes with an antibody which binds to an epitope of a mutant calreticulin protein of SEQ ID NO: 1. Here, the mutant calreticulin protein preferably has an antigen-recognition site in a polypeptide chain consisting of the amino acid sequence of SEQ ID NO: 1 or a polypeptide chain consisting of an amino acid sequence of SEQ ID NO: 1 but having deletion, substitution, or addition of one or several amino acids.

The first domain does not bind to a wild-type calreticulin protein but has a high affinity to the mutant calreticulin protein, and the first domain has a high affinity to the mutant calreticulin protein especially after cleavage.

Examples of cleaved CALR proteins include (A) a polypeptide consisting of the amino acid sequence of SEQ ID NO: 1 and (B) a polypeptide consisting of an amino acid sequence of SEQ ID NO: 1 but having deletion, substitution, or addition of one or several amino acids. In (B), an amino acid sequence in which 1 to 4 amino acids are deleted, substituted, or added is preferred, and an amino acid sequence in which 1 to 3 amino acids are deleted, substituted, or added is more preferred. More specifically, an amino acid sequence in which 3 amino acids are deleted from the C-terminal side of SEQ ID NO: 1 is more preferred. The identity of the amino acid sequence of the polypeptide (B) and the amino acid sequence of SEQ ID NO: 1 is preferably 80% or more, more preferably 85% or more, and even more preferably 90% or more.

The first domain may be any antibody as long as it has an antigen-recognition site (epitope) in the polypeptide chain (A) or (B), and may bind only to a cleaved mutant CALR protein or may be bind to both the cleaved mutant CALR protein and a full-length mutant CALR protein. The antibody is preferably an antibody specific to "mutant CALR protein" which binds to both the cleaved mutant CALR protein and the full-length mutant CALR protein.

Such first domains are preferably heavy-chain and light-chain variable regions of anti-mutant CALR antibodies (clone B3, C6, and G1 antibodies) that the present inventors have found, as well as heavy-chain complementarity determining regions (CDR-H1, CDR-H2, and CDR-H3) and light-chain complementarity determining regions (CDR-L1, CDR-L2, and CDR-L3) or mutants thereof.

The second domain may be a domain which specifically binds to a CD3 antigen and is preferably heavy-chain and light-chain variable regions derived from existing anti-CD3 antibodies, as well as heavy-chain complementarity determining regions (CDR-H1, CDR-H2, and CDR-H3) and light-chain complementarity determining regions (CDR-L1, CDR-L2, and CDR-L3), derived from an already-known anti-CD3 antibody, or mutants thereof. Examples of the existing anti-CD3 antibodies include OKT3.

As preferred examples of the first and second domains, Tables 1-1 to 1-4 show amino acid sequences of heavy-chain and light-chain variable regions of anti-mutant CALR antibodies (clone B3, C6, and G1 antibodies) and mutants thereof and anti-CD3 antibodies (OKT3) and mutants thereof, as well as amino acid sequences of heavy-chain complementarity determining regions (CDR-H1, CDR-H2, and CDR-H3) and light-chain complementarity determining regions (CDR-L1, CDR-L2, and CDR-L3).

**[Table 1-1]**

| SEQ ID NO | Name | Amino acid sequence |
|---|---|---|
| 2 | B3 VH | QVQLQQSGAELVKPGSSVKISCKASGYTFTRNFI HWIKQQPGNGLEWIGWIFPGDGDTEYNQKFNG KATLTADKSSSTAYMQLSSLTSEDSAVYFCARGN YNYEYFDYWGQGVMVTVSS |
| 3 | B3 VL | DIQMTQSPASLSASLGETVSIECLASEDIYSYLA WYQQKPGKSPQLLIFAANRLQDGVPSRFSGSGS GTQFSLKISGMQPEDEGDYFCLQGSKFPYTFGP GTKLELN |
| 4 | C6 VH | QVQLQQSGAELVKPGSSVKISCKASGYTFTSYF VHWIKQQPGDGLEWIGWIYPGDGDTEYNHKFN GKSTLTADRSSSTAYMQLSSLTSEDSAVYFCARG NYYDGREVMDAWGQGASVTVSS |
| 5 | C6VL | DVVLTQTPPTLSATIGQSVSISCRSSQSLLDSDGE TYLNWLLQRPGQSPQLLIYSVSNLESGVPNRFS GSGSETDFTLKISGMEAEDLGVYYCMQATHGP YTFGAGTKLELK |
| 6 | G1 VH | QVQLQQSGAELVKPGSSVKISCKASGYTFTSYF VHWIKQQPGDGLEWIGWIYPGDGDTEYNQKFN GKATLTADRSSSTAYMQLSSLTSEDSAVYFCARG NYYDGREVMDAWGQGASVTVSS |
| 7 | G1 VL | DVVLTQTPPTLSATIGQSVSISCRSSQSLLDSDGE TYLNWLLQRPGQCPQLLIYSVSNLESGVPNRFS GSGSETDFTLKISGVEAEDLGVYYCMQGTHGP YTFGAGTKLELK |
| 8 | hOKT3 VH | QVQLVQSGGGVVQPGRSLRLSCKASGYTFTRY TMHWVRQAPGKGLEWIGYINPSRGYTNYNQK VKDRFTISRDNSKNTAFLQMDSLRPEDTGVYFC ARYYDDHYSLDYWGQGTPVTVSS |
| 204 | hOKT3 VH-2 | QVQLVQSGGGVVQPGRSLRLSCKASGYTFTRY TMHWVRQAPGKGLEWIGYINPSRGYTNYNQK VKDRFTISRDNSKNTAFLQMDSLRPEDTGVYFC ARYYNDHYSLDYWGOGTPVTVSS |
| 205 | hOKT3 VH-3 | QVQLVQSGGGVVQPGRSLRLSCKASGYTFTRY TMHWVRQAPGKGLEWIGYINPSRGYTNYNQK VKDRFTISRDNSKNTAFLQMDSLRPEDTGVYFC ARYYDDHFSLDYWGQGTPVTVSS |
| 206 | hOKT3 VH-4 | QVQLVQSGGGVVQPGRSLRLSCKASGYTFTRY TMHWVRQAPGKGLEWIGYINPSMGATNYNQK VKDRFTISRDNSKNTAFLQMDSLRPEDTGVYFC ARYYDDHYSLDYWGQGTPVTVSS |

**[Table 1-2]**

| SEQ ID NO | Name | Amino acid sequence |
|---|---|---|
| 9 | hOKT3 VL | DIQMTQSPSSLSASVGDRVTITCSASSSVSYMN WYQQTPGKAPKRWIYDTSKLASGVPSRFSGSGS GTDYTFTISSLQPEDIATYYCQQWSSNPFTFGQG TKLQITR |
| 207 | hOKT3 VL-2 | DIQMTQSPSSLSASVGDRVTITCSASSSVAYMN WYQQTPGKAPKRWIYDTSKLASGVPSRFSGSGS GTDYTFTISSLQPEDIATYYCQQWSSNPFTFGQG TKLQITR |
| 208 | hOKT3 VL-3 | DIQMTQSPSSLSASVGDRVTITCSASSSVSYMN WYQQTPGKAPKRWIYNTSKLASGVPSRFSGSGS GTDYTFTISSLQPEDIATYYCQQWSSNPFTFGQG TKLQITR |
| 10 | B3 CDR-H1 | GYTFTRNFIH |
| 11 | B3 CDR-H2 | WIFPGDGDTEYNQKFNG |
| 126 | B3 CDR-H2-2 | WIFPGDADTEYNQKFSG |
| 12 | B3 CDR-H3 | GNYNYEYFDY |
| 13 | B3 CDR-L1 | LASEDIYSYLA |
| 14 | B3 CDR-L2 | AANRLQD |
| 127 | B3 CDR-L2-2 | AANRLQS |
| 15 | B3 CDR-L3 | LQGSKFPYT |
| 16 | C6 CDR-H1 | GYTFTSYFVH |
| 17 | C6 CDR-H2 | WIYPGDGDTEYNHKFNG |
| 18 | C6 CDR-H3 | GNYYDGREVMDA |
| 19 | C6 CDR-L1 | RSSQSLLDSDGETYLN |

**[Table 1-3]**

| SEQ ID NO | Name | Amino acid sequence |
|---|---|---|
| 20 | C6 CDR-L2 | SVSNLES |
| 21 | C6 CDR-L3 | MQATHGPYT |
| 22 | G1 CDR-H1 | GYTFTSYFVH |
| 23 | G1 CDR-H2 | WIYPGDGDTEYNQKFNG |
| 24 | G1 CDR-H3 | GNYYDGREVMDA |
| 25 | G1 CDR-L1 | RSSQSLLDSDGETYLN |
| 26 | G1 CDR-L2 | SVSNLES |
| 27 | G1 CDR-L3 | MQGTHGPYT |
| 28 | OKT3 CDR-H1 | RYTMH |
| 29 | OKT3 CDR-H2 | YINPSRGYTNYNQKVKD |
| 128 | OKT3 CDR-H2-2 | YINPSMGATNYNQKVKD |
| 30 | OKT3 CDR-H3 | YYDDHYSLDY |
| 129 | OKT3 CDR-H3-2 | YYNDHYSLDY |
| 130 | OKT3 CDR-H3-3 | YYDDHFSLDY |
| 31 | OKT3 CDR-L1 | SASSSVSYMN |
| 131 | OKT3 CDR-L1-2 | SASSSVAYMN |

**[Table 1-4]**

| SEQ ID NO | Name | Amino acid sequence |
|---|---|---|
| 32 | OKT3 CDR-L2 | DTSKLAS |
| 132 | OKT3 CDR-L2-2 | NTSKLAS |
| 33 | OKT3 CDR-L3 | QQWSSNPFT |

These regions can be made as recombinant antibodies by genetic engineering techniques based on sequence information of antibodies obtained by immunizing animals with a part or the full length of the cleaved mutant CALR protein or by sensitizing lymphocytes in vitro and then fusing it with myeloma, and antibodies obtained, for example, from antibody libraries such as phage display. The sequence information related to anti-CD3 antibodies can be obtained from existing information. In other words, heavy-chain and light-chain genes of the antibodies are synthesized from the obtained genetic information, inserted into a vector (e.g., plasmid), and then transfected into host cells (e.g., CHO cells, HEK cells, etc.) to culture the host cells, thereby collecting the recombinant antibodies from the culture. In the synthesis of heavy-chain and light-chain genes of the antibodies, it is preferable to optimize codons.

Examples of preferred bispecific antibodies of the present invention include bispecific antibodies in which the CDR of the first domain has (a) an amino acid sequence having 80% or more homology to the amino acid sequences of SEQ ID NO: 10, SEQ ID NO: 11 or 126, and SEQ ID NO: 12 (VHCDR), and an amino acid sequence having 80% or more homology to the amino acid sequences of SEQ ID NO: 13, SEQ ID NO: 14 or 127, and SEQ ID NO: 15 (VLCDR); (b) an amino acid sequence having 80% or more homology to the amino acid sequences of SEQ ID NO: 16 to 18 (VHCDR), and an amino acid sequence having 80% or more homology to the amino acid sequences of SEQ ID NO: 19 to 21 (VLCDR); or (c) an amino acid sequence having 80% or more homology to the amino acid sequences of SEQ ID NO: 22 to 24 (VHCDR), and an amino acid sequence having 80% or more homology to the amino acid sequences of SEQ ID NO: 25 to 27 (VLCDR).

Furthermore, examples thereof include bispecific antibodies in which the first domain has (d) a VH region having an amino acid sequence having 80% or more homology to the amino acid sequences of SEQ ID NO: 2, 90, 91, 92, 93, 94, 133, 134, 135, and 136, and a VL region having an amino acid sequence having 80% or more homology to the amino acid sequences of SEQ ID NO: 3, 95, 96, 97, 98, 99, 137, 138, 139, 140, and 141; (e) a VH region having an amino acid sequence having 80% or more homology to the amino acid sequence of SEQ ID NO: 4, and a VL region having an amino acid sequence having 80% or more homology to the amino acid sequence of SEQ ID NO: 5; or (f) a VH region having an amino acid sequence having 80% or more homology to the amino acid sequence of SEQ ID NO: 6, and a VL region having an amino acid sequence having 80% or more homology to the amino acid sequence of SEQ ID NO: 7.

A bispecific antibody is preferred in which the CDR of the second domain has an amino acid sequence having 80% or more homology to the amino acid sequences of SEQ ID NO: 28, SEQ ID NO: 29 or 128, and SEQ ID NO: 30, 129 or 130 (VHCDR), and an amino acid sequence having 80% or more homology to the amino acid sequences set forth in SEQ ID NO: 31 or 131, SEQ ID NO: 32 or 132, and SEQ ID NO: 33 (VLCDR).

Furthermore, a bispecific antibody is preferred in which the second domain has a VH region having an amino acid sequence having 80% or more homology to the amino acid sequences of SEQ ID NO: 8, 204, 205, and 206, and a VL region having an amino acid sequence having 80% or more homology to the amino acid sequences of SEQ ID NO: 9, SEQ ID NO: 207, and 208.

Examples of more preferred bispecific antibodies of the present invention include bispecific antibodies in which the CDR of the first domain has (a) an amino acid sequence having 80% or more homology to the amino acid sequences of SEQ ID NO: 10, SEQ ID NO: 11 or 126, and SEQ ID NO: 12 (VHCDR), and an amino acid sequence having 80% or more homology to the amino acid sequences of SEQ ID NO: 13, SEQ ID NO: 14 or 127, and SEQ ID NO: 15 (VLCDR); (b) an amino acid sequence having 80% or more homology to the amino acid sequences of SEQ ID NO: 16 to 18 (VHCDR), and an amino acid sequence having 80% or more homology to the amino acid sequences of SEQ ID NO: 19 to 21 (VLCDR); or (c) an amino acid sequence having 80% or more homology to the amino acid sequences of SEQ ID NO: 22 to 24 (VHCDR), and an amino acid sequence having 80% or more homology to the amino acid sequences of SEQ ID NO: 25 to 27 (VLCDR), and in which the CDR of the second domain has an amino acid sequence having 80% or more homology to the amino acid sequences of SEQ ID NO: 28, SEQ ID NO: 29 or 128, and SEQ ID NO: 30, SEQ ID NO: 129 or 130 (VHCDR), and an amino acid sequence having 80% or more homology to the amino acid sequences of SEQ ID NO: 31 or 131, SEQ ID NO: 32 or 132, and SEQ ID NO: 33 (VLCDR).

Examples of further preferred bispecific antibodies include bispecific antibodies in which the first domain has (d) a VH region having an amino acid sequence having 80% or more homology to the amino acid sequences of SEQ ID NO: 2, 90, 91, 92, 93, 94, 133, 134, 135, and 136, and a VL region having an amino acid sequence having 80% or more homology to the amino acid sequences of SEQ ID NO: 3, 95, 96, 97, 98, 99, 137, 138, 139, 140, and 141; (e) a VH region having an amino acid sequence having 80% or more homology to the amino acid sequence of SEQ ID NO: 4, and a VL region having an amino acid sequence having 80% or more homology to the amino acid sequence of SEQ ID NO: 5; or (f) a VH region having an amino acid sequence having 80% or more homology to the amino acid sequence of SEQ ID NO: 6, and a VL region having an amino acid sequence having 80% or more homology to the amino acid sequence of SEQ ID NO: 7, and in which the second domain has a VH region having an amino acid sequence having 80% or more homology to the amino acid sequences of SEQ ID NO: 8, SEQ ID NO: 204, SEQ ID NO: 205, and 206, and a VL region having an amino acid sequence having 80% or more homology to the amino acid sequences of SEQ ID NO: 9, SEQ ID NO: 207, and 208.

As described above, the form of the bispecific antibody of the present invention may be any form as long as it retains specificity for the two antigens, and may be an IgG-like type or a low molecular weight type. Specifically, it may have an Fc region, a hetero-H chain, or the like.

Specific examples of the bispecific antibody include antibodies selected from the group consisting of the following (I) to (XXVII).
(I) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the Fc is further fused to a polypeptide in which the domains are fused via peptide linkers in the order of VH-A, VL-A, VH-B, and VL-B from the N-terminal side; in some cases, the bispecific antibody whose Fc domain is an Fc domain containing one or more amino acid substitutions which reduce the binding to an Fc receptor (Antibody 1: SEQ ID NO: 34).
(II) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the Fc is further fused to a polypeptide in which the domains are fused via peptide linkers in the order of VL-A, VH-A, VH-B, and VL-B from the N-terminal side.
(III) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the Fc is further fused to a polypeptide in which the domains are fused via peptide linkers in the order of VH-B, VL-B, VH-A, and VL-A from the N-terminal side; in some cases, the bispecific antibody whose Fc domain is an Fc domain containing one or more amino acid substitutions which reduce the binding to an Fc receptor (Antibody 3: SEQ ID NO: 36).
(IV) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the Fc is further fused to a polypeptide in which the domains are fused via peptide linkers in the order of VH-B, VL-B, VL-A, and VH-A from the N-terminal side; in some cases, the bispecific antibody whose Fc domain is an Fc domain containing one or more amino acid substitutions which reduce the binding to an Fc receptor (Antibody 4: SEQ ID NO: 37).
(V) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consist of a VH domain containing the amino acid sequences of SEQ ID NO: 28, 29, and 30 (VH-B) and a VL domain containing the amino acid sequences of SEQ ID NO: 31, 32, and 33 (VL-B), wherein the Fc is further fused to a polypeptide in which the domains are fused via peptide linkers in the order of VH-B, VL-A, VH-A, and VL-B from the N-terminal side.
(VI) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the Fc is further fused to a polypeptide in which the domains are fused via peptide linkers in the order of VL-B, VH-A, VL-A, and VH-B from the N-terminal side; in some cases, the bispecific antibody whose Fc domain is an Fc domain containing one or more amino acid substitutions which reduce the binding to an Fc receptor (Antibody 6: SEQ ID NO: 39).
(VII) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the Fc is further fused to a polypeptide in which the domains are fused via peptide linkers in the order of VH-A, VL-B, VH-B, and VL-A from the N-terminal side.
(VIII) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the Fc is further fused to a polypeptide in which the domains are fused via peptide linkers in the order of VL-A, VH-B, VL-B, and VH-A from the N-terminal side.
(IX) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the domains are fused via peptide linkers in the order of VH-A, VL-A, VH-B, and VL-B from the N-terminal side (Antibody 9: SEQ ID NO: 42).
(X) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the domains are fused via peptide linkers in the order of VL-A, VH-A, VH-B, and VL-B from the N-terminal side.
(XI) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the domains are fused via peptide linkers in the order of VH-B, VL-B, VH-A, and VL-A from the N-terminal side (Antibody 11: SEQ ID NO: 44).
(XII) A bispecific antibody, wherein a domain which specifically binds to the mutant CALR protein is a single-chain Fv which consists of a VH domain containing the amino acid sequences of SEQ ID NO: 10, 11, and 12 (VH-A) and a VL domain containing the amino acid sequences of SEQ ID NO: 13, 14, and 15 (VL-A), and a domain which specifically binds to the CD3 antigen is a single-chain Fv which consists of a VH domain containing the amino acid sequences of SEQ ID NO: 28, 29, and 30 (VH-B) and a VL domain containing the amino acid sequences of SEQ ID NO: 31, 32, and 33 (VL-B), wherein the domains are fused via peptide linkers in the order of VH-B, VL-B, VL-A, and VH-A from the N-terminal side.
(XIII) A bispecific antibody, wherein a domain which specifically binds to the mutant CALR protein is an IgG antibody which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, the single-chain Fv being fused to the heavy chain C-terminus of the IgG antibody via a peptide linker, wherein one Fc domain contains a knob and the other Fc domain contains a hole in accordance with the knob-into-hole method, resulting in hetero-association of these antibodies; in some cases, the bispecific antibody whose Fc domain is an Fc domain containing one or more amino acid substitutions which reduce the binding to an Fc receptor (Antibody 13: SEQ ID NO: 46, SEQ ID NO: 47, and SEQ ID NO: 48).
(XIV) A bispecific antibody, wherein a domain which specifically binds to the mutant CALR protein is an IgG antibody which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, the single-chain Fv being fused to the heavy chain C-terminus of the IgG antibody via a peptide linker; in some cases, the bispecific antibody whose Fc domain is an Fc domain containing one or more amino acid substitutions which reduce the binding to an Fc receptor. (Antibody 14: SEQ ID NO: 48 and SEQ ID NO: 49).
(XV) A bispecific antibody, wherein a domain which specifically binds to the mutant CALR protein is an IgG antibody which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33 and the single-chain Fv is fused to the Fc via a peptide linker to form scFv-Fc, wherein one Fc domain contains a knob and the other Fc domain contains a hole in accordance with the knob-into-hole method, resulting in hetero-association of the IgG antibody and the scFv-Fc; in some cases, the bispecific antibody whose Fc domain is an Fc domain containing one or more amino acid substitutions which reduce the binding to an Fc receptor (Antibody 15: SEQ ID NO: 46, SEQ ID NO: 48, and SEQ ID NO: 50).
(XVI) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the single-chain Fv which specifically binds to the CD3 antigen is fused to the heavy chain C-terminus of the Fab fragment which specifically binds to the mutant CALR protein via a peptide linker (Antibody 16: SEQ ID NO: 48 and SEQ ID NO: 51).
(XVII) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the Fc is further fused to a polypeptide in which the single-chain Fv which specifically binds to the CD3 antigen is fused to the heavy chain C-terminus of the Fab fragment which specifically binds to the mutant CALR protein via a peptide linker; in some cases, the bispecific antibody whose Fc domain is an Fc domain containing one or more amino acid substitutions which reduce the binding to an Fc receptor (Antibody 17: SEQ ID NO: 48 and SEQ ID NO: 52) .
(XVIII) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein a CH3 domain is further fused via a peptide linker to a polypeptide in which the single-chain Fv which specifically binds to the CD3 antigen is fused to the heavy chain C-terminus of the Fab fragment which specifically binds to the mutant CALR protein via a peptide linker (Antibody 18: SEQ ID NO: 48 and SEQ ID NO: 53) .
(XIX) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the single-chain Fv which specifically binds to the CD3 antigen is fused to the heavy chain C-terminus of the Fab fragment which specifically binds to the mutant CALR protein via a peptide linker, and the heavy chain N-terminus of the Fab fragment which specifically binds to the mutant CALR protein is further fused to the C-terminus of the single-chain Fv via a peptide linker (Antibody 19: SEQ ID NO: 48 and SEQ ID NO: 54).
(XX) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the heavy chain N-terminus of the Fab fragment which specifically binds to the mutant CALR protein is fused to the C-terminus of the single-chain Fv which specifically binds to the CD3 antigen via a peptide linker (Antibody 20: SEQ ID NO: 48 and SEQ ID NO: 55).
(XXI) A bispecific antibody, wherein a domain which specifically binds to the mutant CALR protein is an IgG antibody which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the heavy chain N-terminus of the IgG which specifically binds to the mutant CALR protein is fused to the C-terminus of the single-chain Fv which specifically binds to the CD3 antigen via a peptide linker (SEQ ID NO: 48 and SEQ ID NO: 56).
(XXII) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen a single-chain Fv which consists of a VH domain containing the amino acid sequences of SEQ ID NO: 28, 29, and 30 and a VL domain containing the amino acid sequences of SEQ ID NO: 31, 32, and 33, wherein a CH3 domain is further fused via peptide linkers to a polypeptide in which the heavy chain N-terminus of the Fab fragment which specifically binds to the mutant CALR protein is fused to the C-terminus of the single-chain Fv which specifically binds to the CD3 antigen via a peptide linker (Antibody 22: SEQ ID NO: 48 and SEQ ID NO: 57).
(XXIII) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the light chain N-terminus of the Fab fragment which specifically binds to the mutant CALR protein is fused to the C-terminus of the single-chain Fv which specifically binds to the CD3 antigen via a peptide linker (Antibody 23: SEQ ID NO: 58 and SEQ ID NO: 59).
(XXIV) A bispecific antibody, wherein a domain which specifically binds to the mutant CALR protein is an IgG antibody which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the light chain N-terminus of the IgG which specifically binds to the mutant CALR protein is fused to the C-terminus of the single-chain Fv which specifically binds to the CD3 antigen via a peptide linker (Antibody 24: SEQ ID NO: 59 and SEQ ID NO: 60).
(XXV) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the bispecific antibody contains a polypeptide in which the light chain N-terminus of the Fab fragment which specifically binds to the mutant CALR protein is fused to the C-terminus of the single-chain Fv which specifically binds to the CD3 antigen via a peptide linker and a polypeptide in which a CH3 domain is further fused to the heavy chain C-terminus of the Fab fragment which specifically binds to the mutant CALR protein via a peptide linker (Antibody 25: SEQ ID NO: 59 and SEQ ID NO: 61) .
(XXVI) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the bispecific antibody contains a polypeptide in which the heavy chain N-terminus of the Fab fragment which specifically binds to the mutant CALR protein is fused to the C-terminus of the single-chain Fv which specifically binds to the CD3 antigen via a peptide linker and a polypeptide in which the light chain N-terminus of the Fab fragment which specifically binds to the mutant CALR protein is fused to the C-terminus of the single-chain Fv which specifically binds to the CD3 antigen via a peptide linker (Antibody 26: SEQ ID NO: 55 and SEQ ID NO: 59).
(XXVII) A bispecific antibody, wherein a domain which specifically binds to the mutant CALR protein is a Fab fragment which consists of a VH domain containing the amino acid sequences of SEQ ID NO: 10, 11, and 12 and a VL domain containing the amino acid sequences of SEQ ID NO: 13, 14, and 15, and a domain which specifically binds to a CD3 antigen is a single-chain Fv which consists of a VH domain containing the amino acid sequences of SEQ ID NO: 28, 29, and 30 and a VL domain containing the amino acid sequences of SEQ ID NO: 31, 32, and 33, wherein the bispecific antibody contains a polypeptide in which the single-chain Fv which specifically binds to the CD3 antigen is fused to the heavy chain C-terminus of the Fab fragment which specifically binds to the mutant CALR protein via a peptide linker and a polypeptide in which the light chain N-terminus of the Fab fragment which specifically binds to the mutant CALR protein is fused to the C-terminus of the single-chain Fv which specifically binds to the CD3 antigen via a peptide linker (Antibody 27: SEQ ID NO: 51 and SEQ ID NO: 59).

**[Table 2]**

| SEQ ID NO | Amino acid sequence |
|---|---|
| 34 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSGGG GSGGGGSGGGGSDIQMTQSPASLSASLGETVSIECLASEDIYSYLAWYQQKPGKSPQLLIF AANRLQDGVPSRFSGSGSGTQFSLKISGMQPEDEGDYFCLQGSKFPYTFGPGTKLELNGGG GSQVQLVQSGGGVVQPGRSLRLSCKASGYTFTRYTMHWVRQAPGKGLEWIGYINPSRGYTN YNQKVKDRFTISRDNSKNTAFLQMDSLRPEDTGVYFCARYYDDHYSLDYWGQGTPVTVSSG GGGSGGGGSGGGGSDIQMTQSPSSLSASVGDRVTITCSASSSVSYMNWYQQTPGKAPKRWI YDTSKLASGVPSRFSGSGSGTDYTFTISSLQPEDIATYYCQQWSSNPFTFGQGTKLQITRD KTHTCPPCPAPEAAGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK |
| 35 | DIQMTQSPASLSASLGETVSIECLASEDIYSYLAWYQQKPGKSPQLLIFAANRLQDGVPSR FSGSGSGTQFSLKISGMQPEDEGDYFCLQGSKFPYTFGPGTKLELNGGGGSGGGGSGGGGS QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSGGG GSQVQLVQSGGGVVQPGRSLRLSCKASGYTFTRYTMHWVRQAPGKGLEWIGYINPSRGYTN YNQKVKDRFTISRDNSKNTAFLQMDSLRPEDTGVYFCARYYDDHYSLDYWGQGTPVTVSSG GGGSGGGGSGGGGSDIQMTQSPSSLSASVGDRVTITCSASSSVSYMNWYQQTPGKAPKRWI YDTSKLASGVPSRFSGSGSGTDYTFTISSLQPEDIATYYCQQWSSNPFTFGQGTKLQITRL EVLFQGPDKTHTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVSNKALPAPIEKT ISKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK |
| 36 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSGGGGSGGGGSDIQMTQSPSSLSASVGDRVTITCSASSSVSYMNWYQQTPGKAPKRWIYD TSKLASGVPSRFSGSGSGTDYTFTISSLQPEDIATYYCQQWSSNPFTFGQGTKLQITRGGG GSQVQLQQSGAELVKPGSSVKISCKASGYTFTRNFIHWIKQQPGNGLEWIGWIFPGDGDTE YNQKFNGKATLTADKSSSTAYMQLSSLTSEDSAVYFCARGNYNYEYFDYWGQGVMVTVSSG GGGSGGGGSGGGGSDIQMTQSPASLSASLGETVSIECLASEDIYSYLAWYQQKPGKSPQLL IFAANRLQDGVPSRFSGSGSGTQFSLKISGMQPEDEGDYFCLQGSKFPYTFGPGTKLELND KTHTCPPCPAPEAAGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK |

**[Table 3]**

| SEQ ID NO | Amino acid sequence |
|---|---|
| 37 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSGGGGSGGGGSDIQMTQSPSSLSASVGDRVTITCSASSSVSYMNWYQQTPGKAPKRWIYD TSKLASGVPSRFSGSGSGTDYTFTISSLQPEDIATYYCQQWSSNPFTFGQGTKLQITRGGG GSDIQMTQSPASLSASLGETVSIECLASEDIYSYLAWYQQKPGKSPQLLIFAANRLQDGVP SRFSGSGSGTQFSLKISGMQPEDEGDYFCLQGSKFPYTFGPGTKLELNGGGGSGGGGSGGG GSQVQLQQSGAELVKPGSSVKISCKASGYTFTRNFIHWIKQQPGNGLEWIGWIFPGDGDTE YNQKFNGKATLTADKSSSTAYMQLSSLTSEDSAVYFCARGNYNYEYFDYWGQGVMVTVSSD KTHTCPPCPAPEAAGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK |
| 38 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSDIQMTQSPASLSASLGETVSIECLASEDIYSYLAWYQQKPGKSPQLLIFAANRLQDGVP SRFSGSGSGTQFSLKISGMQPEDEGDYFCLQGSKFPYTFGPGTKLELNGGGGSGGGGSGGG GSGGGGSQVQLQQSGAELVKPGSSVKISCKASGYTFTRNFIHWIKQQPGNGLEWIGWIFPG DGDTEYNQKFNGKATLTADKSSSTAYMQLSSLTSEDSAVYFCARGNYNYEYFDYWGQGVMV TVSSGGGGSDIQMTQSPSSLSASVGDRVTITCSASSSVSYMNWYQQTPGKAPKRWIYDTSK LASGVPSRFSGSGSGTDYTFTISSLQPEDIATYYCQQWSSNPFTFGQGTKLQITRLEVLFQ GPDKTHTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK |
| 39 | DIQMTQSPSSLSASVGDRVTITCSASSSVSYMNWYQQTPGKAPKRWIYDTSKLASGVPSRF SGSGSGTDYTFTISSLQPEDIATYYCQQWSSNPFTFGQGTKLQITRGGGGSQVQLQQSGAE LVKPGSSVKISCKASGYTFTRNFIHWIKQQPGNGLEWIGWIFPGDGDTEYNQKFNGKATLT ADKSSSTAYMQLSSLTSEDSAVYFCARGNYNYEYFDYWGQGVMVTVSSGGGGSGGGGSGGG GSGGGGSDIQMTQSPASLSASLGETVSIECLASEDIYSYLAWYQQKPGKSPQLLIFAANRL QDGVPSRFSGSGSGTQFSLKISGMQPEDEGDYFCLQGSKFPYTFGPGTKLELNGGGGSQVQ LVQSGGGVVQPGRSLRLSCKASGYTFTRYTMHWVRQAPGKGLEWIGYINPSRGYTNYNQKV KDRFTISRDNSKNTAFLQMDSLRPEDTGVYFCARYYDDHYSLDYWGQGTPVTVSSDKTHTC PPCPAPEAAGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |

**[Table 4]**

| SEQ ID NO | Amino acid sequence |
|---|---|
| 40 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSGGG GSDIQMTQSPSSLSASVGDRVTITCSASSSVSYMNWYQQTPGKAPKRWIYDTSKLASGVPS RFSGSGSGTDYTFTISSLQPEDIATYYCQQWSSNPFTFGQGTKLQITRGGGGSGGGGSGGG GSGGGGSQVQLVQSGGGVVQPGRSLRLSCKASGYTFTRYTMHWVRQAPGKGLEWIGYINPS RGYTNYNQKVKDRFTISRDNSKNTAFLQMDSLRPEDTGVYFCARYYDDHYSLDYWGQGTPV TVSSGGGGSDIQMTQSPASLSASLGETVSIECLASEDIYSYLAWYQQKPGKSPQLLIFAAN RLQDGVPSRFSGSGSGTQFSLKISGMQPEDEGDYFCLQGSKFPYTFGPGTKLELNDKTHTC PPCPAPEAAGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 41 | DIQMTQSPASLSASLGETVSIECLASEDIYSYLAWYQQKPGKSPQLLIFAANRLQDGVPSR FSGSGSGTQFSLKISGMQPEDEGDYFCLQGSKFPYTFGPGTKLELNGGGGSQVQLVQSGGG VVQPGRSLRLSCKASGYTFTRYTMHWVRQAPGKGLEWIGYINPSRGYTNYNQKVKDRFTIS RDNSKNTAFLQMDSLRPEDTGVYFCARYYDDHYSLDYWGQGTPVTVSSGGGGSGGGGSGGG GSGGGGSDIQMTQSPSSLSASVGDRVTITCSASSSVSYMNWYQQTPGKAPKRWIYDTSKLA SGVPSRFSGSGSGTDYTFTISSLQPEDIATYYCQQWSSNPFTFGQGTKLQITRGGGGSQVQ LQQSGAELVKPGSSVKISCKASGYTFTRNFIHWIKQQPGNGLEWIGWIFPGDGDTEYNQKF NGKATLTADKSSSTAYMQLSSLTSEDSAVYFCARGNYNYEYFDYWGQGVMVTVSSLEVLFQ GPDKTHTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK |
| 42 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSGGG GSGGGGSGGGGSDIQMTQSPASLSASLGETVSIECLASEDIYSYLAWYQQKPGKSPQLLIF AANRLQDGVPSRFSGSGSGTQFSLKISGMQPEDEGDYFCLQGSKFPYTFGPGTKLELNGGG GSQVQLVQSGGGVVQPGRSLRLSCKASGYTFTRYTMHWVRQAPGKGLEWIGYINPSRGYTN YNQKVKDRFTISRDNSKNTAFLQMDSLRPEDTGVYFCARYYDDHYSLDYWGQGTPVTVSSG GGGSGGGGSGGGGSDIQMTQSPSSLSASVGDRVTITCSASSSVSYMNWYQQTPGKAPKRWI YDTSKLASGVPSRFSGSGSGTDYTFTISSLQPEDIATYYCQQWSSNPFTFGQGTKLQITRH HHHHH |
| 43 | DIQMTQSPASLSASLGETVSIECLASEDIYSYLAWYQQKPGKSPQLLIFAANRLQDGVPSR FSGSGSGTQFSLKISGMQPEDEGDYFCLQGSKFPYTFGPGTKLELNGGGGSGGGGSGGGGS QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSGGG GSQVQLVQSGGGVVQPGRSLRLSCKASGYTFTRYTMHWVRQAPGKGLEWIGYINPSRGYTN YNQKVKDRFTISRDNSKNTAFLQMDSLRPEDTGVYFCARYYDDHYSLDYWGQGTPVTVSSG GGGSGGGGSGGGGSDIQMTQSPSSLSASVGDRVTITCSASSSVSYMNWYQQTPGKAPKRWI YDTSKLASGVPSRFSGSGSGTDYTFTISSLQPEDIATYYCQQWSSNPFTFGQGTKLQITRH HHHHH |

**[Table 5]**

| SEQ ID NO | Amino acid sequence |
|---|---|
| 44 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSGGGGSGGGGSDIQMTQSPSSLSASVGDRVTITCSASSSVSYMNWYQQTPGKAPKRWIYD TSKLASGVPSRFSGSGSGTDYTFTISSLQPEDIATYYCQQWSSNPFTFGQGTKLQITRGGG GSQVQLQQSGAELVKPGSSVKISCKASGYTFTRNFIHWIKQQPGNGLEWIGWIFPGDGDTE YNQKFNGKATLTADKSSSTAYMQLSSLTSEDSAVYFCARGNYNYEYFDYWGQGVMVTVSSG GGGSGGGGSGGGGSDIQMTQSPASLSASLGETVSIECLASEDIYSYLAWYQQKPGKSPQLL IFAANRLQDGVPSRFSGSGSGTQFSLKISGMQPEDEGDYFCLQGSKFPYTFGPGTKLELNH HHHHH |
| 45 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSGGGGSGGGGSDIQMTQSPSSLSASVGDRVTITCSASSSVSYMNWYQQTPGKAPKRWIYD TSKLASGVPSRFSGSGSGTDYTFTISSLQPEDIATYYCQQWSSNPFTFGQGTKLQITRGGG GSDIQMTQSPASLSASLGETVSIECLASEDIYSYLAWYQQKPGKSPQLLIFAANRLQDGVP SRFSGSGSGTQFSLKISGMQPEDEGDYFCLQGSKFPYTFGPGTKLELNGGGGSGGGGSGGG GSQVQLQQSGAELVKPGSSVKISCKASGYTFTRNFIHWIKQQPGNGLEWIGWIFPGDGDTE YNQKFNGKATLTADKSSSTAYMQLSSLTSEDSAVYFCARGNYNYEYFDYWGQGVMVTVSSH HHHHH |
| 46 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCDKTHTCPPCPAPEAAGGPSVFL FPPKPKDTLMISRTPEVTCVVVDVSHEDPEVKFNWYVDGVEVHNAKTKPREEQYNSTYRVV SVLTVLHQDWLNGKEYKCKVSNKALPAPIEKTISKAKGQPREPQVYTLPPCREEMTKNQVS LWCLVKGFYPSDIAVEWESNGQPENNYKTTPPVLDSDGSFFLYSKLTVDKSRWQQGNVFSC SVMHEALHNHYTQKSLSLSPGK |
| 47 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCDKTHTCPPCPAPEAAGGPSVFL FPPKPKDTLMISRTPEVTCVVVDVSHEDPEVKFNWYVDGVEVHNAKTKPREEQYNSTYRVV SVLTVLHQDWLNGKEYKCKVSNKALPAPIEKTISKAKGQPREPQVCTLPPSREEMTKNQVS LSCAVKGFYPSDIAVEWESNGQPENNYKTTPPVLDSDGSFFLVSKLTVDKSRWQQGNVFSC SVMHEALHNHYTQKSLSLSPGKGGGGSQVQLVQSGGGVVQPGRSLRLSCKASGYTFTRYTM HWVRQAPGKGLEWIGYINPSRGYTNYNQKVKDRFTISRDNSKNTAFLQMDSLRPEDTGVYF CARYYDDHYSLDYWGQGTPVTVSSGGGGSGGGGSGGGGSDIQMTQSPSSLSASVGDRVTIT CSASSSVSYMNWYQQTPGKAPKRWIYDTSKLASGVPSRFSGSGSGTDYTFTISSLQPEDIA TYYCQQWSSNPFTFGQGTKLQITR |
| 48 | DIQMTQSPASLSASLGETVSIECLASEDIYSYLAWYQQKPGKSPQLLIFAANRLQDGVPSR FSGSGSGTQFSLKISGMQPEDEGDYFCLQGSKFPYTFGPGTKLELNRTVAAPSVFIFPPSD EQLKSGTASVVCLLNNFYPREAKVQWKVDNALQSGNSQESVTEQDSKDSTYSLSSTLTLSK ADYEKHKVYACEVTHQGLSSPVTKSFNRGEC |

**[Table 6]**

| | |
|---|---|
| | |
| 49 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCDKTHTCPPCPAPEAAGGPSVFL FPPKPKDTLMISRTPEVTCVVVDVSHEDPEVKFNWYVDGVEVHNAKTKPREEQYNSTYRVV SVLTVLHQDWLNGKEYKCKVSNKALPAPIEKTISKAKGQPREPQVYTLPPSREEMTKNQVS LTCLVKGFYPSDIAVEWESNGQPENNYKTTPPVLDSDGSFFLYSKLTVDKSRWQQGNVFSC SVMHEALHNHYTQKSLSLSPGKGGGGSQVQLVQSGGGVVQPGRSLRLSCKASGYTFTRYTM HWVRQAPGKGLEWIGYINPSRGYTNYNQKVKDRFTISRDNSKNTAFLQMDSLRPEDTGVYF CARYYDDHYSLDYWGQGTPVTVSSGGGGSGGGGSGGGGSDIQMTQSPSSLSASVGDRVTIT CSASSSVSYMNWYQQTPGKAPKRWIYDTSKLASGVPSRFSGSGSGTDYTFTISSLQPEDIA TYYCQQWSSNPFTFGQGTKLQITR |
| 50 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSGGGGSGGGGSDIQMTQSPSSLSASVGDRVTITCSASSSVSYMNWYQQTPGKAPKRWIYD TSKLASGVPSRFSGSGSGTDYTFTISSLQPEDIATYYCQQWSSNPFTFGQGTKLQITREPK SCDKTHTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAK GQPREPQVCTLPPSREEMTKNQVSLSCAVKGFYPSDIAVEWESNGQPENNYKTTPPVLDSD GSFFLVSKLTVDKSRWQQGNVFSCSVMHEALHNHYTQKSLSLSPGK |
| 51 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCGGGGSQVQLVQSGGGVVQPGRS LRLSCKASGYTFTRYTMHWVRQAPGKGLEWIGYINPSRGYTNYNQKVKDRFTISRDNSKNT AFLQMDSLRPEDTGVYFCARYYDDHYSLDYWGQGTPVTVSSGGGGSGGGGSGGGGSDIQMT QSPSSLSASVGDRVTITCSASSSVSYMNWYQQTPGKAPKRWIYDTSKLASGVPSRFSGSGS GTDYTFTISSLQPEDIATYYCQQWSSNPFTFGQGTKLQITRHHHHHH |
| 52 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCGGGGSQVQLVQSGGGVVQPGRS LRLSCKASGYTFTRYTMHWVRQAPGKGLEWIGYINPSRGYTNYNQKVKDRFTISRDNSKNT AFLQMDSLRPEDTGVYFCARYYDDHYSLDYWGQGTPVTVSSGGGGSGGGGSGGGGSDIQMT QSPSSLSASVGDRVTITCSASSSVSYMNWYQQTPGKAPKRWIYDTSKLASGVPSRFSGSGS GTDYTFTISSLQPEDIATYYCQQWSSNPFTFGQGTKLQITRDKTHTCPPCPAPEAAGGPSV FLFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLTVDKSRWQQGNVF SCSVMHEALHNHYTQKSLSLSPGK |

**[Table 7]**

| SEQ ID NO | Amino acid sequence |
|---|---|
| 53 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCGGGGSQVQLVQSGGGVVQPGRS LRLSCKASGYTFTRYTMHWVRQAPGKGLEWIGYINPSRGYTNYNQKVKDRFTISRDNSKNT AFLQMDSLRPEDTGVYFCARYYDDHYSLDYWGQGTPVTVSSGGGGSGGGGSGGGGSDIQMT QSPSSLSASVGDRVTITCSASSSVSYMNWYQQTPGKAPKRWIYDTSKLASGVPSRFSGSGS GTDYTFTISSLQPEDIATYYCQQWSSNPFTFGQGTKLQITRAAAGGGQPREPQVYTLPPSR EEMTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 54 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCGGGGSQVQLVQSGGGVVQPGRS LRLSCKASGYTFTRYTMHWVRQAPGKGLEWIGYINPSRGYTNYNQKVKDRFTISRDNSKNT AFLQMDSLRPEDTGVYFCARYYDDHYSLDYWGQGTPVTVSSGGGGSGGGGSGGGGSDIQMT QSPSSLSASVGDRVTITCSASSSVSYMNWYQQTPGKAPKRWIYDTSKLASGVPSRFSGSGS GTDYTFTISSLQPEDIATYYCQQWSSNPFTFGQGTKLQITRGGGGSQVQLQQSGAELVKPG SSVKISCKASGYTFTRNFIHWIKQQPGNGLEWIGWIFPGDGDTEYNQKFNGKATLTADKSS STAYMQLSSLTSEDSAVYFCARGNYNYEYFDYWGQGVMVTVSSASTKGPSVFPLAPSSKST SGGTAALGCLVKDYFPEPVTVSWNSGALTSGVHTFPAVLQSSGLYSLSSVVTVPSSSLGTQ TYICNVNHKPSNTKVDKRVEPKSCHHHHHH |
| 55 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSGGGGSGGGGSDIQMTQSPSSLSASVGDRVTITCSASSSVSYMNWYQQTPGKAPKRWIYD TSKLASGVPSRFSGSGSGTDYTFTISSLQPEDIATYYCQQWSSNPFTFGQGTKLQITRGGG GSQVQLQQSGAELVKPGSSVKISCKASGYTFTRNFIHWIKQQPGNGLEWIGWIFPGDGDTE YNQKFNGKATLTADKSSSTAYMQLSSLTSEDSAVYFCARGNYNEYFDYWGQGVMVTVSSA STKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKRVEPKSCHHHHHH |
| 56 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSGGGGSGGGGSDIQMTQSPSSLSASVGDRVTITCSASSSVSYMNWYQQTPGKAPKRWIYD TSKLASGVPSRFSGSGSGTDYTFTISSLQPEDIATYYCQQWSSNPFTFGQGTKLQITRGGG GSQVQLQQSGAELVKPGSSVKISCKASGYTFTRNFIHWIKQQPGNGLEWIGWIFPGDGDTE YNQKFNGKATLTADKSSSTAYMQLSSLTSEDSAVYFCARGNYNYEYFDYWGQGVMVTVSSA STKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKRVEPKSCDKTHTCPPCPAPEAAGGPSV FLFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLTVDKSRWQQGNVF SCSVMHEALHNHYTQKSLSLSPGK |

**[Table 8]**

| SEQ ID NO | Amino acid sequence |
|---|---|
| 57 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSGGGGSGGGGSDIQMTQSPSSLSASVGDRVTITCSASSSVSYMNWYQQTPGKAPKRWIYD TSKLASGVPSRFSGSGSGTDYTFTISSLQPEDIATYYCQQWSSNPFTFGQGTKLQITRGGG GSQVQLQQSGAELVKPGSSVKISCKASGYTFTRNFIHWIKQQPGNGLEWIGWIFPGDGDTE YNQKFNGKATLTADKSSSTAYMQLSSLTSEDSAVYFCARGNYNYEYFDYWGQGVMVTVSSA STKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKRVEPKSCAAAGGGQPREPQVYTLPPSR EEMTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 58 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCHHHHHH |
| 59 | QVQLVQSGGGVVQPGRSLRLSCKASGYTFTRYTMHWVRQAPGKGLEWIGYINPSRGYTNYN QKVKDRFTISRDNSKNTAFLQMDSLRPEDTGVYFCARYYDDHYSLDYWGQGTPVTVSSGGG GSGGGGSGGGGSDIQMTQSPSSLSASVGDRVTITCSASSSVSYMNWYQQTPGKAPKRWIYD TSKLASGVPSRFSGSGSGTDYTFTISSLQPEDIATYYCQQWSSNPFTFGQGTKLQITRGGG GSDIQMTQSPASLSASLGETVSIECLASEDIYSYLAWYQQKPGKSPQLLIFAANRLQDGVP SRFSGSGSGTQFSLKISGMQPEDEGDYFCLQGSKFPYTFGPGTKLELNRTVAAPSVFIFPP SDEQLKSGTASVVCLLNNFYPREAKVQWKVDNALQSGNSQESVTEQDSKDSTYSLSSTLTL SKADYEKHKVYACEVTHQGLSSPVTKSFNRGEC |
| 60 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCDKTHTCPPCPAPEAAGGPSVFL FPPKPKDTLMISRTPEVTCVVVDVSHEDPEVKFNWYVDGVEVHNAKTKPREEQYNSTYRVV SVLTVLHQDWLNGKEYKCKVSNKALPAPIEKTISKAKGQPREPQVYTLPPSREEMTKNQVS LTCLVKGFYPSDIAVEWESNGQPENNYKTTPPVLDSDGSFFLYSKLTVDKSRWQQGNVFSC SVMHEALHNHYTQKSLSLSPGK |
| 61 | QVQLQQSGAELVKPGSSVKISCKASGYTFTRNFIHWIKQQPGNGLEWIGWIFPGDGDTEYN QKFNGKATLTADKSSSTAYMQLSSLTSEDSAVYFCARGNYNYEYFDYWGQGVMVTVSSAST KGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCAAAGGGQPREPQVYTLPPSREE MTKNQVSLTCLVKGFYPSDIAVEWESNGQPENNYKTTPPVLDSDGSFFLYSKLTVDKSRWQ QGNVFSCSVMHEALHNHYTQKSLSLSPGK |

The bispecific antibody is preferably an antibody further selected from the group consisting of the following (XXVIII) to (LIV).
(XXVIII)A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the Fc is further fused to a polypeptide in which the domains are fused via peptide linkers in the order of VH-A, VL-A, VH-B, and VL-B from the N-terminal side; in some cases, the bispecific antibody whose Fc domain is an Fc domain containing one or more amino acid substitutions which reduce the binding to an Fc receptor (Antibody 1: SEQ ID NO: 34).
(XXIX) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the Fc is further fused to a polypeptide in which the domains are fused via peptide linkers in the order of VL-A, VH-A, VH-B, and VL-B from the N-terminal side.
(XXX) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the Fc is further fused to a polypeptide in which the domains are fused via peptide linkers in the order of VH-B, VL-B, VH-A, and VL-A from the N-terminal side; in some cases, the bispecific antibody whose Fc domain is an Fc domain containing one or more amino acid substitutions which reduce the binding to an Fc receptor (Antibody 3: SEQ ID NO: 36).
(XXXI) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the Fc is further fused to a polypeptide in which the domains are fused via peptide linkers in the order of VH-B, VL-B, VL-A, and VH-A from the N-terminal side; in some cases, a bispecific antibody whose Fc domain is an Fc domain containing one or more amino acid substitutions which reduce binding to an Fc receptor (Antibody 4: SEQ ID NO: 37).
(XXXII) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the Fc is further fused to a polypeptide in which the domains are fused via peptide linkers in the order of VH-B, VL-A, VH-A, and VL-B from the N-terminal side.
(XXXIII) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the Fc is further fused to a polypeptide in which the domains are fused via peptide linkers in the order of VL-B, VH-A, VL-A, and VH-B from the N-terminal side; in some cases, the bispecific antibody whose Fc domain is an Fc domain containing one or more amino acid substitutions which reduce the binding to an Fc receptor (Antibody 6: SEQ ID NO: 39).
(XXXIV) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the Fc is further fused to a polypeptide in which the domains are fused via peptide linkers in the order of VH-A, VL-B, VH-B, and VL-A from the N-terminal side.
(XXXV) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the Fc is further fused to a polypeptide in which the domains are fused via peptide linkers in the order of VL-A, VH-B, VL-B, and VH-A from the N-terminal side.
(XXXVI) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the domains are fused via peptide linkers in the order of VH-A, VL-A, VH-B, and VL-B from the N-terminal side (Antibody 9: SEQ ID NO: 42).
(XXXVII) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the domains are fused via peptide linkers in the order of VL-A, VH-A, VH-B, and VL-B from the N-terminal side.
(XXXVIII)A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a single-chain Fv which consists of a VH domain of SEQ ID NO: 8 (VH-B) and a VL domain of SEQ ID NO: 9 (VL-B), wherein the domains are fused via peptide linkers in the order of VH-B, VL-B, VH-A, and VL-A from the N-terminal side (Antibody 11: SEQ ID NO: 44).
(XXXIX) A bispecific antibody, wherein a domain which specifically binds to the mutant CALR protein is a single-chain Fv which consists of a VH domain of SEQ ID NO: 2 (VH-A) and a VL domain of SEQ ID NO: 3 (VL-A), and a domain which specifically binds to the CD3 antigen is a VH domain of SEQ ID NO: 8 (VH-B) and a VL domain of SEQ ID NO: 9 (VL-B), wherein the domains are fused via peptide linkers in the order of VH-B, VL-B, VL-A, and VH-A from the N-terminal side.
(XL) A bispecific antibody, wherein a domain which specifically binds to the mutant CALR protein is an IgG antibody which consists of a VH domain of SEQ ID NO: 2 and a VL domain of SEQ ID NO: 3, and a domain which specifically binds to the CD3 antigen is a single -chain Fv which consists of a VH domain of SEQ ID NO: 8 and a VL domain of SEQ ID NO: 9, wherein the single-chain Fv is fused to the heavy chain C-terminus of the IgG antibody via a peptide linker, wherein one Fc domain contains a knob and the other Fc domain contains a hole in accordance with the knob-into-hole method, resulting in hetero-association of these antibodies; in some cases, the bispecific antibody whose Fc domain is an Fc domain containing one or more amino acid substitutions which reduce the binding to an Fc receptor (Antibody 13: SEQ ID NO: 46, SEQ ID NO: 47, and SEQ ID NO: 48).
(XLI) A bispecific antibody, wherein a domain which specifically binds to the mutant CALR protein is an IgG antibody which consists of a VH domain of SEQ ID NO:2 and a VL domain of SEQ ID NO:3, and a domain which specifically binds to the CD3 antigen is a single-chain Fv which consists of VH domain of SEQ ID NO:8 and a VL domain of SEQ ID NO: 9, wherein the single-chain Fv is fused to the heavy chain C-terminus of the IgG antibody via a peptide linker; in some cases, the bispecific antibody whose Fc domain is an Fc domain containing one or more amino acid substitutions which reduce the binding to an Fc receptor (Antibody 14: SEQ ID NO: 48 and SEQ ID NO: 49).
(XLII) A bispecific antibody, wherein a domain which specifically binds to the mutant CALR protein is an IgG antibody which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the single-chain Fv is fused to the Fc via a peptide linker to form scFV-Fc, wherein one Fc domain contains a knob and the other Fc domain contains a hole in accordance with the knob-into-hole method, resulting in hetero-association of the IgG antibody and the scFv-Fc; in some cases, the bispecific antibody whose Fc domain is an Fc domain containing one or more amino acid substitutions which reduce the binding to an Fc receptor (Antibody 15: SEQ ID NO: 46, SEQ ID NO: 48, and SEQ ID NO: 50).
(XLIII) A bispecific antibody, wherein a domain which specifically binds to the mutant CALR protein is a Fab fragment which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the single-chain Fv which specifically binds to the CD3 antigen is fused to the heavy chain C-terminus of the Fab fragment which specifically binds to the mutant CALR protein via a peptide linker (Antibody 16: SEQ ID NO: 48 and SEQ ID NO: 51) .
(XLIV) A bispecific antibody, wherein a domain which specifically binds to the mutant CALR protein is a Fab fragment which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the Fc is further fused to a polypeptide in which the single-chain Fv which specifically binds to the CD3 antigen is fused to the heavy chain C-terminus of the Fab fragment which specifically binds to the mutant CALR protein via a peptide linker; in some cases, the bispecific antibody whose Fc domain is an Fc domain containing one or more amino acid substitutions which reduce the binding to an Fc receptor (Antibody 17: SEQ ID NO: 48 and SEQ ID NO: 52) .
(XLV) A bispecific antibody, wherein a domain which specifically binds to the mutant CALR protein is a Fab fragment which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein a CH3 domain is fused further via a peptide linker to a polypeptide in which the single-chain Fv which specifically binds to the CD3 antigen is fused to the heavy chain C-terminus of the Fab fragment which specifically binds to the mutant CALR protein via a peptide linker (Antibody 18: SEQ ID NO: 48 and SEQ ID NO: 53).
(XLVI) A bispecific antibody, wherein a domain which specifically binds to the mutant CALR protein is a Fab fragment which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the single-chain Fv which specifically binds to the CD3 antigen is fused to the heavy chain C-terminus of the Fab fragment which specifically binds to the mutant CALR protein via a peptide linker, and the heavy chain N-terminus of the Fab fragment which specifically binds to the mutant CALR protein is further fused to the C-terminus of the single-chain Fv via a peptide linker (Antibody 19: SEQ ID NO: 48 and SEQ ID NO: 54).
(XLVII) A bispecific antibody, wherein a domain which specifically binds to the mutant CALR protein is a Fab fragment which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the heavy chain N-terminus of the Fab fragment which specifically binds to the mutant CALR protein is fused to the C-terminus of the single-chain Fv which specifically binds to the CD3 antigen via a peptide linker (Antibody 20: SEQ ID NO: 48 and SEQ ID NO: 55).
(XLVIII) A bispecific antibody, wherein a domain which specifically binds to the mutant CALR protein is an IgG antibody which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the heavy chain N-terminus of the IgG which specifically binds to the mutant CALR protein is fused to the C-terminus of the single-chain Fv which specifically binds to the CD3 antigen via a peptide linker (SEQ ID NO: 48, SEQ ID NO: 56) .
(XLIX) A bispecific antibody, wherein a domain which specifically binds to the mutant CALR protein is a Fab fragment which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein a CH3 domain is fused further via a peptide linker to a polypeptide in which the heavy chain N-terminus of the Fab fragment which specifically binds to the mutant CALR protein is fused to the C-terminus of the single-chain Fv which specifically binds to the CD3 antigen via a peptide linker (Antibody 22: SEQ ID NO: 48 and SEQ ID NO: 57).
(L) A bispecific antibody, wherein a domain which specifically binds to the mutant CALR protein is a Fab fragment which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the light chain N-terminus of the Fab fragment which specifically binds to the mutant CALR protein is fused to the C-terminus of the single-chain Fv which specifically binds to the CD3 antigen via a peptide linker (Antibody 23: SEQ ID NO: 58 and SEQ ID NO: 59).
(LI) A bispecific antibody, wherein a domain which specifically binds to the mutant CALR protein is an IgG antibody which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the light chain N-terminus of the IgG which specifically binds to the mutant CALR protein is fused to the C-terminus of the single-chain Fv which specifically binds to the CD3 antigen via a peptide linker (Antibody 24: SEQ ID NO: 59 and SEQ ID NO: 60).
(LII) A bispecific antibody, wherein a domain which specifically binds to the mutant CALR protein is a Fab fragment which consists of a VH domain of SEQ ID NO: 2 and a VL domain of SEQ ID NO: 3, and a a domain which specifically binds to the CD3 antigen is single-chain Fv which consists of a VH domain of SEQ ID NO: 8 and a VL domain of SEQ ID NO: 9, wherein the bispecific antibody contains a polypeptide in which the light chain N-terminus of the Fab fragment which specifically binds to the mutant CALR protein is fused to the C-terminus of the single-chain Fv which specifically binds to the CD3 antigen via a peptide linker and a polypeptide in which a CH3 domain is further fused to the heavy chain C-terminus of the Fab fragment which specifically binds to the mutant CALR protein via a peptide linker (Antibody 25: SEQ ID NO: 59 and SEQ ID NO: 61).
(LIII) A bispecific antibody, wherein a domain which specifically binds to the mutant CALR protein is a Fab fragment which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the bispecific antibody contains a polypeptide in which the heavy chain N-terminus of the Fab fragment which specifically binds to the mutant CALR protein is fused to the C-terminus of the single-chain Fv which specifically binds to the CD3 antigen via a peptide linker and a polypeptide in which the light chain N-terminus of the Fab fragment which specifically binds to the mutant CALR protein is fused to the C-terminus of the single-chain Fv which specifically binds to the CD3 antigen via a peptide linker (Antibody 26: SEQ ID NO: 55 and SEQ ID NO: 59).
(LIV) A bispecific antibody, wherein a domain which specifically binds to the mutant CALR protein is a Fab fragment which consists of a VH domain of SEQ ID NO: 2 and a VL domain of SEQ ID NO: 3, and a domain which specifically binds to the CD3 antigen is a single-chain Fv which consists of a VH domain of SEQ ID NO: 8 and a VL domain of SEQ ID NO: 9, wherein the bispecific antibody contains a polypeptide in which the single-chain Fv which specifically binds to the CD3 antigen is fused to the heavy chain C-terminus of the Fab fragment which specifically binds to the mutant CALR protein via a peptide linker and a polypeptide in which the light chain N-terminus of the Fab fragment which specifically binds to the mutant CALR protein is fused to the C-terminus of the single-chain Fv which specifically binds to the CD3 antigen via a peptide linker (Antibody 27: SEQ ID NO: 51 and SEQ ID NO: 59).

**[Table 9]**

| Amino acid sequence | Nucleotide sequence |
|---|---|
| SEQ ID NO 34 | SEQ ID NO 62 |
| SEQ ID NO 35 | SEQ ID NO 63 |
| SEQ ID NO 36 | SEQ ID NO 64 |
| SEQ ID NO 37 | SEQ ID NO 65 |
| SEQ ID NO 38 | SEQ ID NO 66 |
| SEQ ID NO 39 | SEQ ID NO 67 |
| SEQ ID NO 40 | SEQ ID NO 68 |
| SEQ ID NO 41 | SEQ ID NO 69 |
| SEQ ID NO 42 | SEQ ID NO 70 |
| SEQ ID NO 43 | SEQ ID NO 71 |
| SEQ ID NO 44 | SEQ ID NO 72 |
| SEQ ID NO 45 | SEQ ID NO 73 |
| SEQ ID NO 46 | SEQ ID NO 74 |
| SEQ ID NO 47 | SEQ ID NO 75 |
| SEQ ID NO 48 | SEQ ID NO 76 |
| SEQ ID NO 49 | SEQ ID NO 77 |
| SEQ ID NO 50 | SEQ ID NO 78 |
| SEQ ID NO 51 | SEQ ID NO 79 |
| SEQ ID NO 52 | SEQ ID NO 80 |
| SEQ ID NO 53 | SEQ ID NO 81 |
| SEQ ID NO 54 | SEQ ID NO 82 |
| SEQ ID NO 55 | SEQ ID NO 83 |
| SEQ ID NO 56 | SEQ ID NO 84 |
| SEQ ID NO 57 | SEQ ID NO 85 |
| SEQ ID NO 58 | SEQ ID NO 86 |
| SEQ ID NO 59 | SEQ ID NO 87 |
| SEQ ID NO 60 | SEQ ID NO 88 |
| SEQ ID NO 61 | SEQ ID NO 89 |

Preferred specific examples of the bispecific antibodies of the present invention include those having each of the (I) to (XXVII) sequences or each of the (XXVIII) to (LIV) sequences, and amino acid sequences in the regions other than these regions are not particularly restricted. The antibody of the present invention may be a humanized antibody and a non-human mammalian antibody. More specifically, the antibody may be a chimeric antibody composed of the variable regions of the heavy chain and light chain of an antibody of a non-human mammal, for example, a rat, and the constant regions of the heavy chain and light chain of a human antibody, and such an antibody can be obtained by ligating a DNA encoding the variable region of a rat antibody with a DNA encoding the constant region of a human antibody, incorporating this into an expression vector and transfecting the vector into a host for production. As an example of the humanized antibody, VH 1 to 8 and VL 1 to 7 are shown below (Tables 10-1 to 10-2 and Table 11).

**[Table 10-1]**

| SEQ ID NO | Name | Amino acid sequence |
|---|---|---|
| 90 | VH1 | QVQLVQSGAEVKKPGASVKVSCKASGYTFTRNFIHWVRQAP GQGLEWMGWIFPGDGDTEYNQKFNGRVTMTRDTSISTAYME LSRLRSDDTAVYYCARGNYNYEYFDYWGQGTMVTVSS |
| 91 | VH2 | QVQLQESGAEVKKPGASVKVSCKASGYTFTRNFIHWVRQAP GQRLEWMGWIFPGDGDTEYNQKFNGRVTITRDTSASTAYME LSSLRSEDTAVYYCARGNYNYEYFDYWGQGTLVTVSS |
| 92 | VH3 | QVQLVQSGAEVKKPGASVKISCKASGYTFTRNFIHWVRQAPG QSLEWVGWIFPGDGDTEYNQKFNGRITITRDTSANTAYMELS SLKSEDTAVYYCARGNYNYEYFDYWGQGTLVTVSS |
| 93 | VH4 | QMQLVQSGAEVKKTGSSVKVSCKASGYTFTRNFIHWVRQAP GQALEWMGWIFPGDGDTEYNQKFNGRVTITRDRSMSTAYME LSSLRSEDTAMYYCARGNYNYEYFDYWGQGTLVTVSS |
| 133 | VH4-2 | QMQLVQSGAEVKKTGSSVKVSCKASGYTFTRNFIHWVRQAP GQALEWMGWIFPGDADTEYNQKFSGRVTITRDRSMSTAYME LSSLRSEDTAMYYCARGNYNYEYFDYWGQGTLVTVSS |
| 94 | VH5 | QVQLVQSGAEVKKPGSSVKVSCKASGYTFTRNFIHWVRQQP GQGLEWMGWIFPGDGDTEYNQKFNGRVTITADKSTSTAYMQ LSSLTSEDTAVYFCARGNYNYEYFDYWGQGVMVTVSS |
| 134 | VH6 | QVQLVQSGAEVKKPGSSVKVSCKASGYTFTRNFIHWIRQAPG QGLEWIGWIFPGDADTEYNQKFSGRATITADKSTSTAYMELSS LRSEDTAVYYCARGNYNYEYFDYWGQGTMVTVSS |
| 135 | VH7 | QVQLVQSGAEVKKPGSSVKVSCKASGYTFTRNFIHWIRQAPG QGLEWIGWIFPGDADTEYNQKFSGRVTITADKSTSTAYMELSS LRSEDTAVYYCARGNYNYEYFDYWGQGTMVTVSS |
| 136 | VH8 | QVQLVQSGAEVKKPGSSVKVSCKASGYTFTRNFIHWIRQAPG QGLEWIGWIFPGDGDTEYNQKFNGRATITADKSTSTAYMELSS LRSEDTAVYYCARGNYNYEYFDYWGQGTMVTVSS |
| 95 | VL1 | DIQMTQSPSFVSASVGDRVTITCLASEDIYSYLAWYQQKPGKA PKLLIFAANRLQDGVPSRFSGGGSGTGFTLTISSLQPEDFATYF CLQGSKFPYTFGQGTKLEIN |
| 137 | VL1-2 | DIQMTQSPSFVSASVGDRVTITCLASEDIYSYLAWYQQKPGKA PKLLIFAANRLQSGVPSRFSGGGSGTGFTLTISSLQPEDFATYFC LQGSKFPYTFGQGTKLEIN |
| 138 | VL1-3 | DIQMTQSPSFVSASVGDRVTITCLASEDIYSYLAWYQQKPGKA PKLLIFAANRLQSGVPSRFSGGGSGTGFTLTISSLQPEDFATYFC LQGSKFPYTFGQGTKLEIK |
| 139 | VL1-4 | DIQMTQSPSFVSASVGDRVTITCLASEDIYSYLAWYQQKPGKA PKLLIFAANRLQDGVPSRFSGGGSGTGFTLTISSLQPEDFATYF CLQGSKFPYTFGQGTKLEIK |
| 96 | VL2 | DIQLTQSPAFLSASLGDRVTITCLASEDIYSYLAWYQQKPGKA PKLLISAANRLQDGVPSRFSGSGSGTEFTLTISSLQPEDFATYSC LQGSKFPYTFGQGTKLEIK |
| 97 | VL3 | DIQLTQSPSSLSASVGDRVTITCLASEDIYSYLAWYQQKPGKG PKLLIYAANRLQDGVPSRFSGSGSGTDFSLTISSLQPEDLATYY CLQGSKFPYTFGGGTKVEIK |
| 98 | VL4 | DIQMTQSPSSLSASVGDTVTIACLASEDIYSYLAWYQQKPGK APKLLIYAANRLQDGVPSRFSGSGSFTDFTLTINSLQPDDSATY YCLQGSKFPYTFGGGTKVDIK |

**[Table 10-2]**

| SEQ ID NO | Name | Amino acid sequence |
|---|---|---|
| 99 | VL5 | DIQMTQSPSSVSASVGDSVTIECLASEDIYSYLAWYQQKPGKS PKLLIFAANRLQDGVPSRFSGSGSGTQFTLKISSLQPEDFAEYF CLQASKFPYTFGPGTKLELN |
| 140 | VL6 | DIQMTQSPSSLSASVGDRVTITCLASEDIYSYLAWYQQKPGKA PKLLIFAANRLQDGVPSRFSGSGSGTDFTLTISSLQPEDFATYY CLQGSKFPYTFGPGTKVDIK |
| 141 | VL7 | DIQMTQSPSSLSASVGDRVTITCLASEDIYSYLAWYQQKPGKA PKLLIFAANRLQSGVPSRFSGSGSGTDFTLTISSLQPEDFATYYC LQGSKFPYTFGPGTKVDIK |

**[Table 11]**

| Amino acid sequence | Nucleotide sequence |
|---|---|
| SEQ ID NO 90 | SEQ ID NO 100 |
| SEQ ID NO 91 | SEQ ID NO 101 |
| SEQ ID NO 92 | SEQ ID NO 102 |
| SEQ ID NO 93 | SEQ ID NO 103 |
| SEQ ID NO 94 | SEQ ID NO 104 |
| SEQ ID NO 95 | SEQ ID NO 105 |
| SEQ ID NO 96 | SEQ ID NO 106 |
| SEQ ID NO 97 | SEQ ID NO 107 |
| SEQ ID NO 98 | SEQ ID NO 108 |
| SEQ ID NO 99 | SEQ ID NO 109 |
| SEQ ID NO 133 | SEQ ID NO 142 |
| SEQ ID NO 134 | SEQ ID NO 143 |
| SEQ ID NO 135 | SEQ ID NO 144 |
| SEQ ID NO 136 | SEQ ID NO 145 |
| SEQ ID NO 137 | SEQ ID NO 146 |
| SEQ ID NO 138 | SEQ ID NO 147 |
| SEQ ID NO 139 | SEQ ID NO 148 |
| SEQ ID NO 140 | SEQ ID NO 149 |
| SEQ ID NO 141 | SEQ ID NO 150 |

Specific examples of bispecific antibodies using humanized antibodies include, but are not limited to, the following sequences (Table 12).

**[Table 12]**

| | SEQ ID NO 116 | SEQ ID NO 117 | SEQ ID NO 118 | SEQ ID NO 119 | SEQ ID NO 120 |
|---|---|---|---|---|---|
| SEQ ID NO 121 | Antibody 31 | Antibody 32 | Antibody 33 | Antibody 34 | Antibody 35 |
| SEQ ID NO 122 | Antibody 36 | Antibody 37 | Antibody 38 | Antibody 39 | Antibody 40 |
| SEQ ID NO 123 | Antibody 41 | Antibody 42 | Antibody 43 | Antibody 44 | Antibody 45 |
| SEQ ID NO 124 | Antibody 46 | Antibody 47 | Antibody 48 | Antibody 49 | Antibody 50 |
| SEQ ID NO 125 | Antibody 51 | Antibody 52 | Antibody 53 | Antibody 54 | Antibody 55 |

Furthermore, specific examples of the humanized bispecific antibodies include the following antibodies (Tables 13-1 to 13-2) .
[A] A bispecific antibody having Structure 1 in Figure 6, wherein a domain which specifically binds to the mutant CALR protein consists of a VH domain containing the amino acid sequences of SEQ ID NO: 10, SEQ ID NO: 11 or 126, and SEQ ID NO: 12 (VH-A) and a VL domain containing the amino acid sequences of SEQ ID NO: 13, SEQ ID NO: 14 or 127, and SEQ ID NO: 15 (VL-A), and a domain which specifically binds to the CD3 antigen consists of a VH domain containing the amino acid sequences of SEQ ID NO: 28, SEQ ID NO: 29 or 128, and SEQ ID NO: 30, 129 or 130 (VH-B) and a VL domain containing the amino acid sequences of SEQ ID NO: 31 or 131, SEQ ID NO: 32 or 132, and SEQ ID NO: 33 (VL-B) (Antibodies 56 to 99) .
[B] A bispecific antibody having Structure 2 in Figure 6, wherein a domain which specifically binds to the mutant CALR protein consists of a VH domain containing the amino acid sequences of SEQ ID NO: 10, SEQ ID NO: 11 or 126, and SEQ ID NO: 12 (VH-A) and a VL domain containing the amino acid sequences of SEQ ID NO: 13, SEQ ID NO: 14 or 127, and SEQ ID NO: 15 (VL-A), and a domain which specifically binds to the CD3 antigen consists of a VH domain containing the amino acid sequences of SEQ ID NO: 28, SEQ ID NO: 29 or 128, and SEQ ID NO: 30, 129 or 130 (VH-B) and a VL domain containing the amino acid sequences of SEQ ID NO: 31 or 131, SEQ ID NO: 32 or 132, and SEQ ID NO: 33 (VL-B) (Antibodies 100 to 104) .
[C] A bispecific antibody having Structure 3 in Figure 6, wherein a domain which specifically binds to the mutant CALR protein consists of a VH domain containing the amino acid sequences of SEQ ID NO: 10, SEQ ID NO: 11 or 126, and SEQ ID NO: 12 (VH-A) and a VL domain containing the amino acid sequences of SEQ ID NO: 13, SEQ ID NO: 14 or 127, and SEQ ID NO: 15 (VL-A), and a domain which specifically binds to the CD3 antigen consisting of a VH domain containing the amino acid sequences of SEQ ID NO: 28, SEQ ID NO: 29 or 128, and SEQ ID NO: 30, 129 or 130 (VH-B) and a VL domain containing the amino acid sequences of SEQ ID NO: 31 or 131, SEQ ID NO: 32 or 132, and SEQ ID NO: 33 (VL-B) (Antibody 105).
[D] A bispecific antibody having Structure 4 in Figure 6, wherein a domain which specifically binds to the mutant CALR protein consists of a VH domain containing the amino acid sequences set forth in SEQ ID NO: 10, SEQ ID NO: 11 or 126, and SEQ ID NO: 12 (VH-A) and a VL domain containing the amino acid sequences of SEQ ID NO: 13, SEQ ID NO: 14 or 127, and SEQ ID NO: 15 (VL-A), and a domain which specifically binds to the CD3 antigen consists of a VH domain containing the amino acid sequences of SEQ ID NO: 28, SEQ ID NO: 29 or 128, and SEQ ID NO: 30, 129 or 130 (VH-B) and a VL domain containing the amino acid sequences of SEQ ID NO: 31 or 131, SEQ ID NO: 32 or 132, and SEQ ID NO: 33 (VL-B) (Antibody 106).
[E] A bispecific antibody having Structure 5 in Figure 6, wherein a domain which specifically binds to the mutant CALR protein consist of a VH domain containing the amino acid sequences of SEQ ID NO: 10, SEQ ID NO: 11 or 126, and SEQ ID NO: 12 (VH-A) and a VL domain containing the amino acid sequences of SEQ ID NO: 13, SEQ ID NO: 14 or 127, and SEQ ID NO: 15 (VL-A), and a domain which specifically binds to the CD3 antigen consists of a VH domain containing the amino acid sequences of SEQ ID NO: 28, SEQ ID NO: 29 or 128, and SEQ ID NO: 30, 129 or 130 (VH-B) and a VL domain containing the amino acid sequences of SEQ ID NO: 31 or 131, SEQ ID NO: 32 or 132, and SEQ ID NO: 33 (VL-B) (Antibodies 107 and 109 to 111).
[F] A bispecific antibody having Structure 6 in Figure 6, wherein a domain which specifically binds to the mutant CALR protein consists of a VH domain containing the amino acid sequences of SEQ ID NO: 10, SEQ ID NO: 11 or 126, and SEQ ID NO: 12 (VH-A) and a VL domain containing the amino acid sequences of SEQ ID NO: 13, SEQ ID NO: 14 or 127, and SEQ ID NO: 15 (VL-A), and a domain which specifically binds to the CD3 antigen consists of a VH domain containing the amino acid sequences of SEQ ID NO: 28, SEQ ID NO: 29 or 128, and SEQ ID NO: 30, 129 or 130 (VH-B) and a VL domain containing the amino acid sequences of SEQ ID NO: 31 or 131, SEQ ID NO: 32 or 132, and SEQ ID NO: 33 (VL-B) (Antibodies 112 and 113).
[G] A bispecific antibody having Structure 7 in Figure 6, wherein a domain which specifically binds to the mutant CALR protein consists of a VH domain containing the amino acid sequences of SEQ ID NO: 10, SEQ ID NO: 11 or 126, and SEQ ID NO: 12 (VH-A) and a VL domain containing the amino acid sequences of SEQ ID NO: 13, SEQ ID NO: 14 or 127, and SEQ ID NO: 15 (VL-A), and a domain which specifically binds to the CD3 antigen consists of a VH domain containing the amino acid sequences of SEQ ID NO: 28, SEQ ID NO: 29 or 128, and SEQ ID NO: 30, 129 or 130 (VH-B) and a VL domain containing the amino acid sequences of SEQ ID NO: 31 or 131, SEQ ID NO: 32 or 132, and SEQ ID NO: 33 (VL-B) (Antibody 114).

**[Table 13-1]**

| Name | Structure | Amino acid sequence 1 | Amino acid sequence 2 | Amino acid sequence 3 |
|---|---|---|---|---|
| Antibody 56 | Structure 1 | SEQ ID NO: 151 | SEQ ID NO: 121 | |
| Antibody 57 | Structure 1 | SEQ ID NO: 152 | SEQ ID NO: 121 | |
| Antibody 58 | Structure 1 | SEQ ID NO: 153 | SEQ ID NO: 121 | |
| Antibody 59 | Structure 1 | SEQ ID NO: 154 | SEQ ID NO: 121 | |
| Antibody 60 | Structure 1 | SEQ ID NO: 155 | SEQ ID NO: 121 | |
| Antibody 61 | Structure 1 | SEQ ID NO: 156 | SEQ ID NO: 121 | |
| Antibody 62 | Structure 1 | SEQ ID NO: 157 | SEQ ID NO: 121 | |
| Antibody 63 | Structure 1 | SEQ ID NO: 158 | SEQ ID NO: 121 | |
| Antibody 64 | Structure 1 | SEQ ID NO: 159 | SEQ ID NO: 121 | |
| Antibody 65 | Structure 1 | SEQ ID NO: 160 | SEQ ID NO: 121 | |
| Antibody 66 | Structure 1 | SEQ ID NO: 161 | SEQ ID NO: 121 | |
| Antibody 67 | Structure 1 | SEQ ID NO: 162 | SEQ ID NO: 121 | |
| Antibody 68 | Structure 1 | SEQ ID NO: 157 | SEQ ID NO: 165 | |
| Antibody 69 | Structure 1 | SEQ ID NO: 158 | SEQ ID NO: 165 | |
| Antibody 70 | Structure 1 | SEQ ID NO: 159 | SEQ ID NO: 165 | |
| Antibody 71 | Structure 1 | SEQ ID NO: 160 | SEQ ID NO: 165 | |
| Antibody 72 | Structure 1 | SEQ ID NO: 161 | SEQ ID NO: 165 | |
| Antibody 73 | Structure 1 | SEQ ID NO: 162 | SEQ ID NO: 165 | |
| Antibody 74 | Structure 1 | SEQ ID NO: 157 | SEQ ID NO: 167 | |
| Antibody 75 | Structure 1 | SEQ ID NO: 158 | SEQ ID NO: 167 | |
| Antibody 76 | Structure 1 | SEQ ID NO: 159 | SEQ ID NO: 167 | |
| Antibody 77 | Structure 1 | SEQ ID NO: 160 | SEQ ID NO: 167 | |
| Antibody 78 | Structure 1 | SEQ ID NO: 161 | SEQ ID NO: 167 | |
| Antibody 79 | Structure 1 | SEQ ID NO: 162 | SEQ ID NO: 166 | |
| Antibody 80 | Structure 1 | SEQ ID NO: 170 | SEQ ID NO: 197 | |
| Antibody 81 | Structure 1 | SEQ ID NO: 175 | SEQ ID NO: 197 | |
| Antibody 82 | Structure 1 | SEQ ID NO: 170 | SEQ ID NO: 198 | |
| Antibody 83 | Structure 1 | SEQ ID NO: 171 | SEQ ID NO: 198 | |
| Antibody 84 | Structure 1 | SEQ ID NO: 172 | SEQ ID NO: 198 | |
| Antibody 85 | Structure 1 | SEQ ID NO: 173 | SEQ ID NO: 198 | |
| Antibody 86 | Structure 1 | SEQ ID NO: 174 | SEQ ID NO: 198 | |

**[Table 13-2]**

| | | | | |
|---|---|---|---|---|
| Antibody 87 | Structure 1 | SEQ ID NO: 175 | SEQ ID NO: 198 | |
| Antibody 88 | Structure 1 | SEQ ID NO: 176 | SEQ ID NO: 198 | |
| Antibody 89 | Structure 1 | SEQ ID NO: 177 | SEQ ID NO: 198 | |
| Antibody 90 | Structure 1 | SEQ ID NO: 178 | SEQ ID NO: 198 | |
| Antibody 91 | Structure 1 | SEQ ID NO: 179 | SEQ ID NO: 198 | |
| Antibody 92 | Structure 1 | SEQ ID NO: 180 | SEQ ID NO: 198 | |
| Antibody 93 | Structure 1 | SEQ ID NO: 181 | SEQ ID NO: 198 | |
| Antibody 94 | Structure 1 | SEQ ID NO: 182 | SEQ ID NO: 197 | |
| Antibody 95 | Structure 1 | SEQ ID NO: 183 | SEQ ID NO: 197 | |
| Antibody 96 | Structure 1 | SEQ ID NO: 184 | SEQ ID NO: 197 | |
| Antibody 97 | Structure 1 | SEQ ID NO: 185 | SEQ ID NO: 197 | |
| Antibody 98 | Structure 1 | SEQ ID NO: 186 | SEQ ID NO: 197 | |
| Antibody 99 | Structure 1 | SEQ ID NO: 187 | SEQ ID NO: 197 | |
| Antibody 100 | Structure 2 | SEQ ID NO: 163 | SEQ ID NO: 168 | |
| Antibody 101 | Structure 2 | SEQ ID NO: 164 | SEQ ID NO: 168 | |
| Antibody 102 | Structure 2 | SEQ ID NO: 164 | SEQ ID NO: 169 | |
| Antibody 103 | Structure 2 | SEQ ID NO: 189 | SEQ ID NO: 199 | |
| Antibody 104 | Structure 2 | SEQ ID NO: 190 | SEQ ID NO: 199 | |
| Antibody 105 | Structure 3 | SEQ ID NO: 188 | | |
| Antibody 106 | Structure 4 | SEQ ID NO: 194 | SEQ ID NO: 197 | |
| Antibody 107 | Structure 5 | SEQ ID NO: 191 | SEQ ID NO: 197 | SEQ ID NO: 200 |
| Antibody 109 | Structure 5 | SEQ ID NO: 193 | SEQ ID NO: 198 | SEQ ID NO: 200 |
| Antibody 110: | Structure 5 | SEQ ID NO: 212 | SEQ ID NO: 198 | SEQ ID NO: 200 |
| Antibody 111 | Structure 5 | SEQ ID NO: 191 | SEQ ID NO: 198 | SEQ ID: NO200 |
| Antibody 112 | Structure 6 | SEQ ID NO: 195 | SEQ ID NO: 203 | SEQ ID NO: 202 |
| Antibody 113 | Structure 6 | SEQ ID NO: 195 | SEQ ID NO: 203 | SEQ ID NO: 199 |
| Antibody 114 | Structure 7 | SEQ ID NO: 196 | SEQ ID NO: 201 | |

Another method for obtaining human antibodies is also known. For example, human lymphocytes are sensitized with a desired antigen or a cell expressing a desired antigen in vitro, and the sensitized lymphocytes are fused with human myeloma cells, such as U266, to obtain a desired human antibody having a binding activity to the antigen (See JP-B-1-59878). A desired human antibody can be obtained by immunizing a transgenic animal having all repertories of human antibody genes with a desired antigen (See WO93/12227, WO92/03918, WO94/02602, WO94/25585, WO96/34096, and WO96/33735). Furthermore, a technique of obtaining a human antibody by panning using a human antibody library is also known. For example, a variable region of a human antibody is expressed as a single-chain antibody (scFv) on the surface of the phage by the phage display method, and the phage binding to the antigen can be selected. By analyzing the gene of the selected phage, the DNA sequence encoding the variable region of the human antibody which binds to the antigen can be determined. If the DNA sequence of the scFv which binds to the antigen is revealed, a human antibody can be obtained by making an appropriate expression vector with the sequence. These methods are already well known, and WO92/01047, WO92/20791, WO93/06213, WO93/11236, WO93/19172, WO95/01438, and WO95/15388 can be referred to.

For the bispecific antibodies, bispecific antibody expression vectors can be constructed using, for example, DNA encoding anti-mutant CALR and anti-CD3 antibodies and expression vectors, and the expression vectors can be transfected into CHO cells or HEK293 cells to obtain transformed cells. The culture solution of these transformed cells can be purified by chromatography. The method for making the bispecific antibodies are already well known, and JP2019022497, JP2017137329, and JP2015110628 can be referred to.

The class of the antibody is not particularly limited, and antibodies having any isotypes, such as IgG, IgM, IgA, IgD, or IgE, are included. In consideration of, for example, the ease of purification, IgG is preferred.

Examples of domain structures include low molecular weight antibodies, such as antibody fragments, and modified antibodies. Specific examples of the antibody fragments include, but are not limited to, Fab, Fab', F(ab')², Fv, scFv, and diabodies. Examples described later show the H chain constant region of human IgG with amino acid mutations at 228, 233-238, 265, 268, 309, 318, and 328-331 which are believed to suppress binding affinity to the Fc receptor and amino acid mutations at 349, 354, 366, 368 and 407 for the hetero H chain, but not exclusively.

Among the antibodies of the present invention, the antibody used for detection of the polypeptide (A) or (B) may be any antibody which binds to these polypeptides. Meanwhile, the antibody used for prevention or treatment of an MPN is preferably an antibody which binds to the polypeptide (A) or (B) and has a cytotoxic activity.

The antibody which binds to the cleaved mutant CALR protein may be any antibody which binds to a polypeptide chain having the sequence of SEQ ID NO: 1, and is preferably an antibody that competes with at least one of the antibodies or functional fragments thereof for binding to an amino acid sequence portion of SEQ ID NO: 1 in the cleaved mutant CALR protein.

The affinity of the mutant CALR-CD3 bispecific antibody to the mutant CALR protein may be a binding constant (K_{D}) of 10⁻⁷ M or less and is preferably 10⁻⁸ M or less. The binding constant is preferably measured by the surface plasmon resonance (SPR) method but can be determined simply by flow cytometry analysis from the concentration dependence of binding to mutant CALR-expressing cells.

Here, examples of the biological sample used for the detection include a sample that is a biological sample collected from a subject and contains the polypeptide (A) or (B). Specifically, examples of the sample include whole blood, plasma, serum, lymphocytes, lymph fluid, platelets, mononuclear cells, granulocytes, body fluids such as saliva and urine, and tissue samples (bone marrow fluid and bone marrow tissue) obtained by bone marrow biopsy.

Examples of the method for detecting the polypeptide (A) or (B) include liquid chromatography-mass spectrometry, an immunological method, and a method using a low or medium molecule having the specific binding ability or nucleic acid, or a nucleic acid derivative, and an immunological method is preferred because of its ease and high measurement sensitivity.

The immunological method is preferably an immunological method using an antibody which has an antigen-recognition site (epitope) in the polypeptide chain (A) or (B), or a functional fragment thereof. The epitope in the polypeptide chain (A) or (B) may be any sequence present in the polypeptide chain (A) or (B), and is preferably a peptide having three or more consecutive amino acids in the polypeptide chain (A) or (B) and more preferably a peptide having five or more consecutive amino acids in the polypeptide chain (A) or (B).

The antibody to be used in the immunological method may be any antibody obtained using a peptide having, for example, three or more consecutive amino acids in the polypeptide chain (A) or (B) as the antigen, and may be a polyclonal antibody, a monoclonal antibody, or a bispecific antibody, and is preferably a bispecific antibody. Here, the monoclonal antibody can be produced by, for example, obtaining antibody-producing cells by sensitization with a peptide having three or more consecutive amino acids in the polypeptide chain (A) or (B), fusing the antibody-producing cells with myeloma cells to prepare hybridomas, selecting a clone producing the target antibody, and proliferating this cell. The bispecific antibody can be produced by constructing a bispecific antibody expression vector using a DNA encoding the antibody and an expression vector, and then proliferating cells transformed with this vector.

As the immunological method, for example, any of immunochromatography, enzyme-labeled immunoassay (EIA), radioimmunoassay (RIA), coagulation method, nephelometry, flow cytometric method, tissue immunostaining, and immunoblotting such as western blotting can be used. Here, immunoblotting is a method for transferring a polypeptide onto a membrane and detecting the polypeptide on the membrane with an antibody. In the detection of the antibody, for example, an enzyme label and a fluorescent label are used.

If the polypeptide (A) or (B) is detected in a biological sample, the biological sample can be determined to be a biological sample derived from an MPN patient.

The agent for diagnosis of MPN of the present invention contains an antibody which has an antigen-recognition site (epitope) in the polypeptide chain (A) or (B). This antibody is preferably an above-described antibody.

The agent for diagnosis of MPN of the present invention may also contain, for example, a buffer and a measurement protocol, in addition to the antibody.

The pharmaceutical composition such as the agent for prevention or treatment of MPN of the present invention is only required to contain the antibody and can be produced by formulation together with a pharmaceutically acceptable carrier through, for example, mixing, dissolving, emulsifying, encapsulating, or lyophilizing.

The MPNs to be prevented or treated with the pharmaceutical composition of the present invention is preferably an MPN from which mutant CALR is detected.

Suitable formulations for oral administration are, for example, liquids prepared by dissolving an effective amount of the antibody of the present invention in diluents such as water and physiological saline; capsules, granules, powders, or tablets containing an effective amount of the antibody as solids or granules; suspensions prepared by suspending an effective amount of the antibody in an appropriate dispersion medium, and emulsions prepared by dispersing and emulsifying, in an appropriate dispersion medium, a solution prepared by dissolving an effective amount of the antibody.

For parenteral administration, the antibody of the present invention can be formulated into dosage forms such as a solution for injection, a suspension, an emulsion, a cream, an ointment, an inhalant, and a suppository together with pharmaceutically acceptable solvents, excipients, binders, stabilizers, dispersants, and the like. In the prescription for injection, the antibody of the present invention can be dissolved in an aqueous solution, preferably a physiologically compatible buffer such as Hanks' solution, Ringer's solution, or physiological saline buffer. Furthermore, the pharmaceutical of the present invention can be in forms such as a suspension, a solution, or an emulsion in an oily or aqueous vehicle. Alternatively, the antibody of the present invention may be produced in a form of powder, and an aqueous solution or a suspension may be prepared using sterile water or the like before use. For administration by inhalation, the antibody of the present invention is powdered, and a powder mixture can be formed with an appropriate base such as lactose or starch. A suppository prescription can be produced by mixing the antibody of the present invention with a commonly used suppository base such as cocoa butter. Furthermore, the therapeutic agent of the present invention can be prescribed as a sustained release formulation by encapsulating the agent in a polymer matrix or the like.

The method for screening for an agent for treatment of MPN of the present invention is characterized by screening for an antibody, a protein, a low or medium molecule, a nucleic acid, or a nucleic acid derivative which recognizes the polypeptide (A) or (B). Specifically, exemplified is the development of an antibody pharmaceutical having an antibody-dependent cytotoxic activity or a complement-dependent cytotoxic activity using an antibody which recognizes the polypeptide (A) or (B). The antibody of the present invention is an example of the antibody pharmaceutical obtained by the screening method of the present invention. Other examples include the development of an antitumor drug that specifically deliver molecules with cytotoxic activity to tumor, for example, a low- or medium molecule, a nucleic acid or nucleic acid derivative, or a protein having a cytocidal effect on tumor cells using the creation of a low- or medium molecule, a nucleic acid or nucleic acid derivative, or a protein which specifically binds to the polypeptide (A) or (B).

### Examples

Next, the present invention will be described in more detail with reference to Examples, but the present invention is not limited to these Examples at all.

### [Test Example 1] Making of antibodies which recognize mutant CALR protein containing cleaved type

A peptide having cysteine added to the C-terminus or N-terminus of the amino acid sequence (RRMMRTKMR) contained in SEQ ID NO: 1 was synthesized and conjugated to a carrier protein, keyhole limpet hemocyanin (KLH), then immunized to 8-week old female WKY/Izm rats. After booster immunization, lymphocytes were collected. The lymphocytes were cell-fused with mouse myeloma SP2 cells by the PEG method, then cultured in a selective medium to obtain hybridomas. The specificity of antibodies in the culture supernatant was screened by the ELISA method using the immunized peptide or purified protein to obtain hybridomas (clones B3, C6, and G1) producing antibodies which bind to mutant CALR proteins containing a cleaved type. Antibodies produced by these hybridomas (clone B3 antibody, C6 antibody, and G1 antibody) were purified and used in tests.

### [Test Example 2] Making of mutant CALR expressing cells

Human megakaryoblastic leukemia cell line UT-7/TPO cells were transfected with a vector expressing a Del 52 or Ins 5 type mutant CALR protein and a vector used for transfection as a control. The resulting UT-7/TPO CALR Del 52 cells and UT-7/TPO CALR Ins 5 cells neoplastically proliferating, and UT-7/TPO vec cells were each seeded at a density of 6.0 × 10⁵ cells/mL in Opti-MEM medium (Thermo Fisher Scientific K.K.) and cultured in the presence of 5% CO₂ at 37°C for 32 hours. On this occasion, UT-7/TPO vec cells were cultured in a medium containing 10 ng/mL TPO.

### [Test Example 3] Verification of recognition of mutant CALR proteins

UT-7/TPO cells expressing a Del 52 type or Ins 5 type mutant CALR having a FLAG-tag inserted downstream of the signal sequence at the N-terminus were cultured, and culture supernatants containing the secreted mutant CALR proteins were prepared by centrifugation (1,600 g × 5 mins, 4°C). The culture supernatants were heat-treated in the presence of SDS and a reducing agent, then a gel by SDS polyacrylamide electrophoresis was performed, after that electrically transferred on a polyvinylidene difluoride (PVDF) membrane, and reacted with a TBS-T solution containing 5% skim milk at room temperature for 1 hour, followed by reaction with a 5% BSA/TBS-T solution containing a rat clone B3, C6, or G1 antibody or mouse anti-DYKDDDDK tag antibody (FUJIFILM Wako Pure Chemical Corporation) at 4°C overnight. After washing with a TBS-T solution, the reaction products were each reacted with a 5% skim milk/TBS-T solution containing a peroxidase-labeled goat anti-rat IgG antibody (Jackson Immuno Research Inc.) or a peroxidase-labeled goat anti-mouse IgG antibody (Jackson Immuno Research Inc.) at room temperature for 1 hour. After washing with a TBS-T solution, the PVDF membranes were reacted with a peroxidase luminescence reagent. The resulting signals were detected using a FUSION image pickup apparatus.

As a result, as shown in Figure 2, it was verified that all of the clone B3, C6, and G1 antibodies recognize the full-length and cleaved mutant CALR proteins.

### [Test Example 4] Determination of sequence information of antibody of the present invention

To obtain the sequence information of heavy-chain and light-chain antibodies of the present invention, mRNAs were prepared from cells producing the antibodies using a PureLinc RNA Mini kit (Thermo Fisher Scientific K.K.). After cDNAs were synthesized using the resulting mRNAs as templates and reverse transcription primers specific to the heavy chain or the light chain by the rapid amplification of cDNA ends method, the cDNAs were cloned into plasmids. The resulting plasmids were analyzed by sanger sequencing to determine the full-length sequences of the cDNAs of the heavy chain and light chain.

### [Example 1] Preparation of bispecific antibodies Material and test method

Bispecific antibody expression vectors were constructed using DNA encoding bispecific antibodies based on the sequence information of clone B3, C6, and G1 antibodies and anti-CD3 antibodies (Tables 1-1 to 1-4) and expression vectors (pcDNA3.4, Thermo Fisher Scientific K.K.). The expression vectors were transfected into CHO cells or HEK293 cells by using an ExpiFectamine CHO Transfection Kit (Thermo Fisher Scientific K.K.) or an ExpiFectamine 293 Transfection Kit (Thermo Fisher Scientific K.K.), transformed cells were cultured, and then the cells were removed by centrifugation and filtration to collect the culture solution. Purification of the antibodies was performed by a combination of affinity chromatography using nickel-conjugated agarose and gel filtration chromatography, a combination of ProteinA affinity chromatography and gel filtration chromatography, or a combination of affinity chromatography using a CaptureSelect kappa XL resin (Thermo Fisher Scientific K.K.) and gel filtration chromatography. In the same manner as above, based on the sequence information described in Patent Literature 4 and Patent Literature 2, bispecific antibodies (Antibody 28: SEQ ID NO: 110, and SEQ ID NO: 111, Antibody 29: SEQ ID NO: 112, and SEQ ID NO: 113, Antibody 30: SEQ ID NO: 114, and SEQ ID NO: 115) and B3 chimeric antibodies (SEQ ID NO: 48 and SEQ ID NO: 60) were prepared.

### [Example 2] Verification of recognition of mutant CALR protein on the cell surface by flow cytometry analysis

### Material and test method

UT-7/TPO vec cells transfected with only the vector used for transfection as a control and mutant CALR-expressing UT-7/TPO CALR Ins 5 cells and UT-7/TPO CALR Del 52 cells were cultured using an IMDM medium containing 10% fetal bovine inactivated serum (FBS) in the presence of 5% CO₂ at 37°C. The 5 × 10⁴ cells in the proliferative stage were centrifuged (400 g × 5 mins, 4°C), and then various antibodies were prepared in an FBS/PBS solution at a concentration of 5 µg/mL and reacted on ice for 30 minutes. After the reaction, the cells were washed twice using a MACS buffer, and an anti-human Fc antibody was prepared as a secondary antibody in an FBS/PBS solution at a concentration of 2.5 µg/mL and reacted on ice for 30 minutes. After completion of the reaction, the cells were washed twice by adding a MACS buffer performing centrifugation (400 g × 5 mins, 4°C), and discarding the supernatant. Signals were quantitatively measured by flow cytometry analysis using BD LSRFortessa X-20 (BD Biosciences).

The results of flow cytometry analysis of [a] UT-7/TPO vec cells, [b] UT-7/TPO CALR Ins 5 cells, and [c] UT-7/TPO CALR Del 52 cells using representative anti-cleaved mutant CALR-CD3 bispecific antibodies made using a VH chain of SEQ ID NO: 2, a VL chain of SEQ ID NO: 3, a VH chain of SEQ ID NO: 8, and a VL chain of SEQ ID NO: 9 are shown.

As a result, as shown in Figure 3, it was verified that the evaluated bispecific antibodies specifically recognize cells expressing mutant CALR proteins on the cell surface.

### [Example 3] Verification of recognition of CD3 antigen on the cell surface by flow cytometry analysis

### Material and test method

Peripheral blood mononuclear cells (PBMC) isolated from healthy human donors were cultured for two days in plates coated with an anti-CD3 antibody (Takara Bio Inc.) from 4 to 24 hours, and then further cultured in an RPMI-1640 medium containing IL-2 and 10% FBS (Sigma-Aldrich) from 10 to 21 days to induce T-LAK cells. The 5 × 10⁵ T-LAK cells were centrifuged (400 g × 5 mins, 4°C), and then anti-cleaved mutant CALR-CD3 bispecific antibodies made using a VH chain of SEQ ID NO: 2, a VL chain of SEQ ID NO: 3, a VH chain of SEQ ID NO: 8, and a VL chain of SEQ ID NO: 9 in an FBS/PBS solution were prepared at a concentration of 5 µg/mL and reacted on ice for 30 minutes. After the reaction, the cells were washed twice using a MACS buffer, and an anti-human Fc antibody was prepared as a secondary antibody in an FBS/PBS solution at a concentration of 5 µg/mL and reacted on ice for 30 minutes. After completion of the reaction, the cells were washed twice by adding a MACS buffer performing centrifugation (400 g × 5 mins, 4°C), and discarding the supernatant. Signals were quantitatively measured by flow cytometry analysis using BD LSRFortessa X-20 (BD Bioscience).

As a result, as shown in Figure 4, it was verified that the bispecific antibodies of the present invention specifically recognize CD3 antigens expressing on the T-LAK cell surface.

### [Example 4] Evaluation of avidity to antigen by surface plasmon resonance analysis

### Material and test method

Sensor chips (GE Healthcare) for surface plasmon resonance analysis were loaded on a surface plasmon resonance analyzer Biacore T200 (GE Healthcare), and then equilibrated with an HBS-EP buffer (GE Healthcare). Anti-cleaved mutant CALR-CD3 bispecific antibodies made using a VH chain of SEQ ID NO: 2, a VL chain of SEQ ID NO: 3, a VH chain of SEQ ID NO: 8, and a VL chain of SEQ ID NO: 9, which were prepared at a concentration of 1 µg/mL with Acetate 5.5 (GE Healthcare) were immobilized using an Amine coupling kit (GE Healthcare) so that the amount of an immobilized antibody was 200 RU. Subsequently, surface plasmon resonance was measured using a peptide having cysteine added to the N-terminus of the amino acid sequence (RRMMRTKMR) contained in SEQ ID NO: 1 prepared at a concentration of 20, 10, 5, 2.5, and 1.25 nM with an HBS-EP buffer, and the dissociation constant was acquired with Biacore T200 Evaluation Software.

As another method, measurements were performed using the molecular interaction analysis system BLItz (ForteBio), which applies biolayer interferometry and surface plasmon resonance technologies. Specifically, a protein having an Fc region and a His-tag added to the N-terminus of SEQ ID NO: 1 was immobilized at a concentration of 0.1 mg/mL using the Anti-Penta-HIS or ProteinA biosensor (ForteBio). Subsequently, the antibodies 1 to 114 made were prepared at a concentration of 500, 100, and 50 nM and reacted to acquire the dissociation constant.

As a result, as shown in Figure 5, Table 14, and Table 16-1 to 16-2, the bispecific antibodies of the present invention strongly bind to the mutant CALR protein.

**[Table 14]**

| Name | K_{D} value (M) | Name | K_{D} value (M) |
|---|---|---|---|
| Antibody 1 | 6.55E-11 | Antibody 19 | 7.28E-10 |
| Antibody 3 | 1.55E-8 | Antibody 20 | 1.24E-8 |
| Antibody 4 | 1.25E-9 | Antibody 22 | <1E-12 |
| Antibody 6 | 1.34E-10 | Antibody 23 | 6.79E-9 |
| Antibody 9 | 6.55E-9 | Antibody 24 | 5.31E-10 |
| Antibody 11 | N.D. | Antibody 25 | N.D. |
| Antibody 13 | <1E-12 | Antibody 26 | 1.23E-8 |
| Antibody 14 | <1E-12 | Antibody 27 | 1.36E-8 |
| Antibody 15 | 6.68E-9 | Antibody 28 (SEQ ID NO: 110 and 111) | 1.48E-3 |
| Antibody 16 | 2.83E-9 | Antibody 29 (SEQ ID NO: 112 and 113) | 5.70E-6 |
| Antibody 17 | <1E-12 | Antibody 30 (SEQ ID NO: 114 and 115) | - |
| Antibody 18 | <1E-12 | B3 chimeric antibody (SEQ ID NO: 48 and 60) | <1E-12 |

| | | | |
|---|---|---|---|
| -: No antibody expression N.D.: Not Determined | | | |

### [Example 5] Evaluation of in vitro cytotoxic activity Material and test method

The cytotoxic activity of the bispecific antibody was measured in a cytolysis assay based on fluorescent dye release. It was verified whether the antibodies of the present invention induce cytolysis of target cells by immune effector cells in vitro. The immune effector cells used were T-LAK cells, but very similar assays can be used for NK cells, macrophages, monocytes, dendritic cells, PBMCs, or the like.

UT-7/TPO CALR Ins 5 cells expressing the mutant CALR were labeled using about 10 µM Calcein-AM (PromoKine) at 37°C for 30 minutes and washed with HBSS three times. After counting the appropriate number of labeled target cells, the cells were placed in 96-well round-bottom culture plates and incubated at 37°C for 2 hours in the presence or absence of various concentrations of bispecific antibodies and control proteins, or in the absence of antibodies added. T-LAK cells were induced by culturing PBMCs isolated from healthy human donors for two days in plates coated with an anti-CD3 antibody (Takara Bio Inc.) from 4 to 24 hours, and further culturing in an RPMI-1640 medium containing IL-2 and 10% FBS from 10 to 21 days. The immune effector cells and labeled target cells were added at a ratio of 10:1. The ratio of other immune effector cells to labeled target cells is also appropriate. Plates containing the labeled target cells, bispecific antibodies, and immune effector cells were cultured at 5% CO₂ from 1 to 6 hours at 37°C. After the reaction for 1 to 6 hours, the cells were centrifuged (125 rpm × 5 mins) to collect the supernatants. Subsequently, the supernatants were placed in 96-well flat-bottom culture plates, and the released dye in the incubation medium was measured for absorbance at 485/526 nm with a fluorescence reader (SynergyH1M, Biotech Japan Corporation). For normalization, the maximum lysis rate (=100%) of labeled target cells was obtained by the addition of a 10 × lysis solution. The minimum lysis rate (=0%) was calculated from wells incubated with the labeled target cells without any constructs or antibodies. The cell cytolysis rate was calculated using the following formula: [(lysis rate of tumor cells in the presence of the bispecific antibody)-(lysis rate of tumor cells in the absence of the bispecific antibody)]/[(maximum lysis rate)-(minimum lysis rate)]. S-shaped dose-response curves were calculated by Prism Software (GraphPad Software Inc., San Diego), and EC50 values (50% effective concentration) were calculated.

As shown in Table 15, the bispecific antibody of the present invention showed cytotoxic activity against UT-7/TPO CALR Ins 5 cells expressing mutant CALR.

**[Table 15]**

| Name | EC50(pM) | Name | EC50(pM) |
|---|---|---|---|
| Antibody 1 | 20 | Antibody 19 | 3714 |
| Antibody 3 | 19 | Antibody 20 | 1463 |
| Antibody 4 | 23 | Antibody 22 | 101 |
| Antibody 6 | 85 | Antibody 23 | 370 |
| Antibody 9 | 1528 | Antibody 24 | 138 |
| Antibody 11 | 1262 | Antibody 25 | 56 |
| Antibody 13 | 4473 | Antibody 26 | 39 |
| Antibody 14 | 989 | Antibody 27 | 36 |
| Antibody 15 | 1290 | Antibody 28 (SEQ ID NO: 110 and 111) | 5433 |
| Antibody 16 | 334 | Antibody 29 (SEQ ID NO: 112 and 113) | 634 |
| Antibody 17 | 46 | Antibody 30 (SEQ ID NO: 114 and 115) | - |
| Antibody 18 | 81 | B3 chimeric antibody (SEQ ID NO: 48 and 60) | >10000 |

| | | | |
|---|---|---|---|
| -: No antibody expression | | | |

As shown in Tables 16-1 to 16-2, the humanized antibodies (Antibody 56 to Antibody 114) were strongly bound to the mutant CALR proteins and showed cytotoxic activity against UT-7/TPO CALR Ins 5 cells expressing the mutant CALR.

**[Table 16-1]**

| Name | Binding affinity KD (M) | Binding affinity EC50 (pM) |
|---|---|---|
| Antibody 56 | <1E-12 | 45 |
| Antibody 57 | <1E-12 | 398 |
| Antibody 58 | <1E-12 | 1531 |
| Antibody 59 | <1E-12 | 469 |
| Antibody 60 | <1E-12 | 77 |
| Antibody 61 | <1E-12 | 9763 |
| Antibody 62 | <1E-12 | 91 |
| Antibody 63 | 2.62E-10 | 197 |
| Antibody 64 | <1E-12 | 829 |
| Antibody 65 | <1E-12 | 397 |
| Antibody 66 | <1E-12 | 115 |
| Antibody 67 | <1E-12 | 5899 |
| Antibody 68 | <1E-12 | 27 |
| Antibody 69 | <1E-12 | 162 |
| Antibody 70 | <1E-12 | 507 |
| Antibody 71 | 5.54E-10 | 448 |
| Antibody 72 | <1E-12 | 93 |
| Antibody 73 | <1E-12 | N.D. |
| Antibody 74 | <1E-12 | 31 |
| Antibody 75 | <1E-12 | 77 |
| Antibody 76 | <1E-12 | 300 |
| Antibody 77 | <1E-12 | 129 |
| Antibody 78 | <1E-12 | 63 |
| Antibody 79 | <1E-12 | N.D. |
| Antibody 80 | <1E-12 | 28 |
| Antibody 81 | <1E-12 | N.D. |
| Antibody 82 | <1E-12 | 22 |
| Antibody 83 | <1E-12 | 110 |
| Antibody 84 | <1E-12 | 277 |
| Antibody 85 | <1E-12 | 389 |

**[Table 16-2]**

| Name | Binding affinity KD (M) | Binding affinity EC50 (pM) |
|---|---|---|
| Antibody 86 | <1E-12 | 58 |
| Antibody 87 | <1E-12 | N.D. |
| Antibody 88 | <1E-12 | 28 |
| Antibody 89 | <1E-12 | 77 |
| Antibody 90 | <1E-12 | 269 |
| Antibody 91 | <1E-12 | 230 |
| Antibody 92 | <1E-12 | 47 |
| Antibody 93 | <1E-12 | N.D. |
| Antibody 94 | <1E-12 | 32 |
| Antibody 95 | <1E-12 | N.D. |
| Antibody 96 | <1E-12 | N.D. |
| Antibody 97 | <1E-12 | N.D. |
| Antibody 98 | <1E-12 | N.D. |
| Antibody 99 | <1E-12 | N.D. |
| Antibody 100 | <1E-12 | 1081 |
| Antibody 101 | <1E-12 | 1664 |
| Antibody 102 | <1E-12 | 850 |
| Antibody 103 | <1E-12 | 399 |
| Antibody 104 | <1E-12 | 621 |
| Antibody 105 | 1.54E-9 | 26 |
| Antibody 106 | 4.99E-9 | 2565 |
| Antibody 107 | 1.21E-8 | 3869 |
| Antibody 109 | 9.91E-9 | 3796 |
| Antibody 110 | 5.33E-9 | 2541 |
| Antibody 111 | 1.28E-8 | 6108 |
| Antibody 112 | <1E-12 | N.D. |
| Antibody 113 | 3.24E-11 | N.D. |
| Antibody 114 | 6.06E-9 | 790 |

| | | |
|---|---|---|
| N.D.: Not Determined | | |

## Claims

1. A bispecific antibody comprising a first domain which specifically binds to a mutant calreticulin protein and a second domain which specifically binds to a CD3 antigen.

2. The bispecific antibody according to claim 1,
wherein the first domain competes with an antibody which binds to an epitope of a mutant calreticulin protein of SEQ ID NO: 1.

3. The bispecific antibody according to claim 1 or 2,
wherein the mutant calreticulin protein has an antigen-recognition site in a polypeptide chain consisting of the amino acid sequence of SEQ ID NO: 1 or a polypeptide chain consisting of an amino acid sequence of SEQ ID NO: 1 but having deletion, substitution, or addition of one or several amino acids.

4. The bispecific antibody according to any one of claims 1 to 3, wherein the first domain does not bind to a wild-type calreticulin protein but has high affinity to the mutant calreticulin protein.

5. The bispecific antibody according to any one of claims 1 to 4, wherein the first domain has a high affinity to a mutant calreticulin protein after cleavage.

6. The bispecific antibody according to any one of claims 1 to 5, wherein binding of the first domain with the mutant calreticulin protein has a K_{D} of 10⁻⁷ M or less.

7. The bispecific antibody according to any one of claims 1 to 6, wherein a CDR of the first domain has (a) an amino acid sequence having 80% or more homology to the amino acid sequences of SEQ ID NO: 10, SEQ ID NO: 11 or 126, and SEQ ID NO: 12 (VHCDR) and an amino acid sequence having 80% or more homology to the amino acid sequences of SEQ ID NO: 13, SEQ ID NO: 14 or 127, and SEQ ID NO: 15 (VLCDR); (b) an amino acid sequence having 80% or more homology to the amino acid sequences of SEQ ID NO: 16 to 18 (VHCDR) and an amino acid sequence having 80% or more homology to the amino acid sequences of SEQ ID NO: 19 to 21 (VLCDR); or (c) an amino acid sequence having 80% or more homology to the amino acid sequences of SEQ ID NO: 22 to 24 (VHCDR) and an amino acid sequence having 80% or more homology to the amino acid sequences of SEQ ID NO: 25 to 27 (VLCDR).

8. The bispecific antibody according to any one of claims 1 to 7, wherein the first domain has (d) a VH region having an amino acid sequence having 80% or more homology to the amino acid sequences of SEQ ID NO: 2, 90, 91, 92, 93, 94, 133, 134, 135, and 136, and a VL region having an amino acid sequence having 80% or more homology to the amino acid sequences of SEQ ID NO: 3, 95, 96, 97, 98, 99, 137, 138, 139, 140, and 141; (e) a VH region having an amino acid sequence having 80% or more homology to the amino acid sequence of SEQ ID NO: 4, and a VL region having an amino acid sequence having 80% or more homology to the amino acid sequence of SEQ ID NO: 5; or (f)a VH region having an amino acid sequence having 80% or more homology to the amino acid sequence of SEQ ID NO: 6, and a VL region having an amino acid sequence having 80% or more homology to the amino acid sequence of SEQ ID NO: 7.

9. The bispecific antibody according to any one of claims 1 to 8, wherein a CDR of the second domain has an amino acid sequence having 80% or more homology to the amino acid sequences of SEQ ID NO: 28, SEQ ID NO: 29 or 128, and SEQ ID NO: 30, 129 or 130 (VHCDR), and an amino acid sequence having 80% or more homology to the amino acid sequences of SEQ ID NO: 31 or 131, SEQ ID NO: 32 or 132, and SEQ ID NO: 33 (VLCDR).

10. The bispecific antibody according to any one of claims 1 to 9, wherein the second domain has a VH region having an amino acid sequence having 80% or more homology to the amino acid sequences of SEQ ID NO: 8, SEQ ID NO: 204, SEQ ID NO: 205, and 206, and a VL region having an amino acid sequence having 80% or more homology to the amino acid sequences of in SEQ ID NO: 9, SEQ ID NO: 207, and 208.

11. The bispecific antibody according to any one of claims 1 to 10, wherein a dimerization module is a hetero-H chain having one or more knob-into-hole structures, wherein an amino acid(s) at one or more positions selected from the group consisting of 349, 354, 366, 368, and 407 in a constant region of the H chain are replaced by Y349C, T366S, L368A, Y407V (hole or cavity), S354C, or T366W (knob or protrusion), the bispecific antibody comprising an Fc region of SEQ ID NO: 209 or 210.

12. The bispecific antibody according to any one of claims 1 to 11, comprising a mutated Fc region of SEQ ID NO: 211 with substitution of an amino acid(s) at one or more positions selected from the group consisting of 234 and 235 in a H-chain Fc region to each introduce L234A or L235A mutation.

13. The bispecific antibody according to any one of claims 1 to 12, which is a human antibody, a humanized antibody, a chimeric antibody of an animal-derived antibody and a human antibody, or a synthetic antibody.

14. A pharmaceutical composition comprising the bispecific antibody according to any one of claims 1 to 13 or a functional fragment thereof.

15. The pharmaceutical composition according to claim 14, which is a pharmaceutical composition for treatment or diagnosis of a myeloproliferative neoplasm.

16. A method for detecting a mutant calreticulin protein, comprising detecting the following polypeptide (A) or (B) in a biological sample using the bispecific antibody according to any one of claims 1 to 13 or a functional fragment thereof:
(A) a polypeptide consisting of the amino acid sequence of SEQ ID NO: 1; or
(B) a polypeptide consisting of the amino acid sequence of SEQ ID NO: 1 but having deletion, substitution, or addition of one or several amino acids.

17. The bispecific antibody according to any one of claims 1 to 13 or a functional fragment thereof, for use in detection of a mutant calreticulin protein.

18. A diagnostic method for a myeloproliferative neoplasm, comprising detecting the following polypeptide (A) or (B) in a biological sample using the bispecific antibody according to any one of claims 1 to 13 or a functional fragment thereof:
(A) a polypeptide consisting of the amino acid sequence of SEQ ID NO: 1; or
(B) a polypeptide consisting of the amino acid sequence of SEQ ID NO: 1 but having deletion, substitution, or addition of one or several amino acids.

19. The bispecific antibody according to any one of claims 1 to 13 or a functional fragment thereof, for use in treatment or diagnosis of a myeloproliferative neoplasm.

20. Use of the bispecific antibody according to any one of claims 1 to 13 or a functional fragment thereof, for producing an agent for treatment or diagnosis of a myeloproliferative neoplasm.

21. A method for prevention or treatment of tumor, comprising administering the bispecific antibody according to any one of claims 1 to 13 or a functional fragment thereof.

22. The method according to claim 21, wherein the tumor is a hematologic tumor, and preferably a myeloproliferative neoplasm, myeloid leukemia, thrombocytosis, polycythemia, primary myelofibrosis, or secondary myelofibrosis.
